# EUROPEAN PATENT APPLICATION

(11) **EP 4 785 958 A1**
(43) Date of publication of application: **05.08.2026**
(21) Application number: 24872530.1
(22) Date of filing: 27.09.2024
(51) Int. Cl.: A61K 45/00, A61K 39/395, A61P 13/10, A61P 21/02, A61P 43/00, C07K 16/28

(54) **PREVENTIVE OR THERAPEUTIC AGENT FOR ASSOCIATED SYMPTOMS ACCOMPANYING DISEASE INDUCED BY SPINAL CORD OR BRAIN DISORDER**

(30) Priority: 29.09.2023 JP 2023170409
(71) Applicant: Tanabe Pharma Corporation, Osaka-shi, Osaka 541-8505 (JP)
(72) Inventor: SASAKI, Atsushi, Osaka-shi, Osaka 541-8505 (JP); KANEKO, Satoshi, Osaka-shi, Osaka 541-8505 (JP); MOGI, Yasuyuki, Osaka-shi, Osaka 541-8505 (JP)
(74) Representative: Hoffmann Eitle
(86) International application number: PCT/JP2024/034720
(87) International publication number: WO 2025/070749

(57) **Abstract**

Provided is a prophylactic or therapeutic agent for associated symptoms accompanying diseases caused by a spinal cord disorder or a brain disorder such as spinal cord injury and HTLV-1 associated myelopathy, comprising an RGMa inhibiting substance. Also provided is a prophylactic or therapeutic agent for spasticity or neurogenic bladder comprising an RGMa inhibiting substance.

## Description

### Technical Field

The present invention relates to a prophylactic or therapeutic agent for associated symptoms accompanying diseases caused by a spinal cord disorder or a brain disorder, comprising an RGMa inhibiting substance.

### Background Art

It is known that after a certain period following central nervous system injury, spinal reflexes are hyperactive, causing excessive muscle contraction and the onset of spasticity. Spasticity is caused not only by injuries such as head trauma and spinal cord injury but also by various central nervous system disorders of the spinal cord and brain, and develops due to causes such as multiple sclerosis, spinocerebellar degeneration, HTLV-1 associated myelopathy (HAM), myelopathy, stroke, brain tumor, cerebral palsy, and hypoxic encephalopathy. There are reports estimating that there are more than 80,000 cases of severe spasticity derived from sequelae of head trauma, spinal cord injury, cerebral palsy, multiple sclerosis, etc., in Japan (Non-patent Document 1), but the exact prevalence of spasticity in Japan is unknown. In overseas reports, about 70% of patients with spinal cord injury develop spasticity (Non-patent Document 2, Non-patent Document 3), which affects rehabilitation and QOL. In addition, it is reported that spasticity develops in 30 to 90% of patients with multiple sclerosis (Non-patent Document 4).

Lesions of the central nervous system (brain or spinal cord) result in upper motor neuron syndrome as positive signs or negative signs. Positive signs include increased muscle tone, exaggerated tendon reflex, radiation of stretch reflexes to other muscles, findings of spasticity such as clonus, spastic dystonia, pathological synkinesis and co-contraction, and hyperactive flexor reflexes; negative signs include paralysis and muscle weakness, and movements lack dexterity and require effort. Although all positive signs of upper motor neuron syndrome are often called spasticity, Lance's definition (Non-patent Document 5) is narrower, stating that spasticity is a positive sign of upper motor neuron syndrome and is a motor disorder characterized by a velocity-dependent increase in tonic stretch reflexes (muscle tone) with exaggerated tendon jerks.

Symptoms of spasticity are diverse and complex depending on the causative disease and the site of the disorder. Spastic paralysis is a notable complication, but since there are not a few cases where the degrees of spasticity and paralysis do not parallel each other, it is suggested that there are differences in the neural mechanisms of the manifestation of both symptoms (Non-patent Document 6). For example, it has been reported that the severity of injury and the degree of spasticity do not match in patients with spinal cord injury and animal models (Non-patent Document 7, Non-patent Document 8).

Furthermore, it takes a certain amount of time from the occurrence of the cause until the onset of spasticity, and as a reason for this, the possibility of the emergence of some neural plasticity or reorganization has been reported (Non-patent Document 9), and the detailed pathophysiology of spasticity has not yet been elucidated.

Physical therapy is the basis for the treatment of spasticity, and muscle relaxants, block therapy, and intrathecal baclofen therapy are considered for severe spasticity (Non-patent Document 10). Specifically, exercise therapy using manual techniques or devices such as standing tables, physical therapy using cold, heat, low-frequency electricity, vibration stimulation, etc., and orthotic therapy are performed for spastic muscles (Non-patent Document 11). When symptoms are severe, drug therapy with muscle relaxant effects may be used. Tizanidine, eperisone, baclofen, diazepam, gabapentin, dantrolene sodium, etc. are used, but it is necessary to note that these drugs have side effects such as drowsiness and muscle weakness (Non-patent Document 12, Non-patent Document 13). If sufficient effects are not obtained with drug treatment, block therapy with botulinum injection into hypertonic muscles has been performed in recent years (Non-patent Document 14, Non-patent Document 15). However, the injection interval must be at least 3 months, and adjustment of the injection dose to obtain a sustained effect is difficult. For severe spasticity, ITB (intrathecal baclofen therapy) is also considered (Non-patent Document 16). A continuous infusion pump capable of continuous administration is experimentally administered into the intrathecal space, and pump implantation is performed only if efficacy is observed.

Thus, existing treatment methods are not necessarily satisfactory as therapeutic agents for spasticity, and there is a strong desire for superior therapeutic agents for spasticity to improve the quality of life (QOL) of patients themselves and by reducing the burden on caregivers.

Neurogenic bladder causing voiding dysfunction and the like is a bladder dysfunction caused by neurological diseases including nerve injury such as spinal cord injury, and lower urinary tract symptoms are observed. Lower urinary tract function is divided into urine storage and voiding, each of which is classified into bladder function and urethral function, and is regulated by cooperative action. That is, during urine storage, intravesical pressure is kept low, the urethra is always closed, and urethral closure pressure is maintained higher than intravesical pressure. On the other hand, during voiding, voiding can be started voluntarily, and the urethra opens along with contraction of the bladder, discharging urine.

Voiding dysfunction is caused by decreased contractility of the detrusor muscle of the bladder or increased urethral resistance, and is accompanied by symptoms such as decreased urine volume, splitting of the urinary stream, interruption of the urinary stream, delayed voiding, abdominal pressure voiding, and terminal dribbling. For treatment, anticholinergic drugs, cholinergic drugs, and α1-adrenergic receptor antagonists exist as drugs indicated for neurogenic bladder in Japan. However, anticholinergic drugs require higher doses than usual to improve neurogenic bladder, and side effects are a problem. α1-adrenergic receptor antagonists and cholinergic drugs have poor evidence, such as not showing clear efficacy in randomized trials, and there are concerns about serious adverse events such as cholinergic crisis. In addition, although the efficacy of β3-adrenergic receptor agonists has been reported, there is no high evidence from randomized trials or the like, and they are currently off-label (Non-patent Document 17, Non-patent Document 18).

From the above, further effective therapeutic agents for neurogenic bladder caused by increased urethral resistance or decreased contractility of bladder smooth muscle, and symptoms thereof, are desired.

RGM (repulsive guidance molecule) is a membrane protein originally identified as an axon guidance molecule in the visual system (Non-patent Document 19). The RGM family includes three members called RGMa, RGMb, and RGMc (Non-patent Document 20), and it is known that at least RGMa and RGMb work with the same signal transduction mechanism (Non-patent Document 21). RGMc exerts an important role in iron metabolism.

Subsequent studies have revealed that RGM has functions such as axon guidance and lamina formation in Xenopus and chick embryos, and regulation of cranial neural tube closure in mouse embryos (Non-patent Document 22). Patent Document 1 discloses an axon regeneration promoting agent comprising an anti-RGM neutralizing antibody as an active ingredient.

In addition to functions in developmental stages, RGMa is re-expressed after central nervous system injury in adult humans and rats, and RGMa inhibition enhances axonal growth after spinal cord injury in rats and promotes functional recovery (Non-patent Document 23), so RGMa is considered to be an inhibitor of axonal regeneration after central nervous system injury. Specific antibodies that neutralize RGMa are described in, for example, Patent Document 2 (e.g., 5F9, 8D1), Patent Document 3 (e.g., AE12-1, AE12-1Y), and Patent Document 4 (e.g., r116A3, r70E4, r116A3C, rH116A3).

Thus, although the role of RGMa in central nervous system injury has been clarified, the involvement of RGMa has not been identified particularly in spasticity and neurogenic bladder, and such therapeutic agents are not known.

### Prior Art Documents

### Patent Documents

Patent Document 1: International Publication WO 2005/087268
Patent Document 2: International Publication WO 2009/106356
Patent Document 3: International Publication WO 2013/112922
Patent Document 4: International Publication WO 2016/175236

### Non-Patent Documents

Non-Patent Document 1: Taira Takaomi, Akagawa Hiroyuki, Okada Yoshikazu et al. Epidemiological survey of spasticity in Japan. Rehabilitation Medicine 2000;37(11):863
Non-Patent Document 2: Archives of Physical Medicine and Rehabilitation 2017;98:1132-8
Non-Patent Document 3: Spinal Cord (2016) 54, 973-979
Non-Patent Document 4: Multiple Sclerosis Journal 2004 Oct;10(5):589-95
Non-Patent Document 5: Spasticity: Disordered Motor Control. Chicago, Year Book Medical Publishers, 1980, pp.485-494
Non-Patent Document 6: Tanaka Reisaku: Neural mechanism of spasticity - Revisited. Rehabilitation Medicine 1995; 32: 97-105
Non-Patent Document 7: J Neurophysiol 128: 470-479, 2022
Non-Patent Document 8: PLoS ONE 12(2): e0171937. doi:10.1371/journal.pone.0171937
Non-Patent Document 9: Nat Neurosci 2004;7:269-277
Non-Patent Document 10: Multiple Sclerosis / Neuromyelitis Optica Clinical Practice Guidelines 2017
Non-Patent Document 11: Expert Review of Neurotherapeutics, 13(12 Suppl), 55-59 (2013)
Non-Patent Document 12: Clinical Neuroscience, 32, 1296-1298 (2014)
Non-Patent Document 13: Neurology, 51(2), 609-611 (1998)
Non-Patent Document 14: Journal of Neurology, 253(suppl 1), i16-i20 (2006)
Non-Patent Document 15: Journal of Neurology, 253(suppl 1), i26-i28 (2006)
Non-Patent Document 16: Journal of Neurosurgery, 21(2), e6 (2006)
Non-Patent Document 17: Journal of Pharmacological Sciences, 112, 121-127 (2010)
Non-Patent Document 18: Clinical Practice Guidelines for Neurogenic Lower Urinary Tract Dysfunction in Spinal Cord Injury, 2019
Non-Patent Document 19: Stahl, B., Muller, B., von Boxberg, Y., Cox, E.C. & Bonhoeffer, F. Biochemical characterization of a putative axonal guidance molecule of the chick visual system. Neuron 5, 735-743 (1990)
Non-Patent Document 20: Mueller et al., Philos. Trans. R. Soc. Lond. B Biol. Sci., 361: 1513-29, 2006
Non-Patent Document 21: Liu, X., Hashimoto, M., Horii, H., Yamaguchi, A., Naito, K. and Yamashita, T. Repulsive guidance molecule b inhibits neurite growth and is increased after spinal cord injury. Biochem. Biophys. Res. Commun. 382, 795-800 (2009)
Non-Patent Document 22: Yamashita, T., Mueller, B.K. & Hata, K. Neogenin and repulsive guidance molecule signaling in the central nervous system. Curr. Opin. Neurobiol.17, 29-34 (2007)
Non-Patent Document 23: Hata, K. et al. RGMa inhibition promotes axonal growth and recovery after spinal cord injury. J. Cell Biol. 173, 47-58 (2006)

### Summary of Invention

### Problem to be Solved by the Invention

An object of the present invention is to provide an effective drug for associated symptoms of diseases caused by a spinal cord disorder or a brain disorder.

### Means for Solving the Problem

As a result of intensive studies to solve the above problems, the present inventors have found that an RGMa inhibiting substance, particularly an anti-RGMa neutralizing antibody, can be expected to have a therapeutic or prophylactic effect on associated symptoms of diseases caused by a spinal cord disorder or a brain disorder, specifically diseases caused by injury or inflammation of the spinal cord or brain such as spinal cord injury and HTLV (Human T-lymphotropic Virus)-1 associated myelopathy, and have completed the present invention.

The present invention is as follows.
1. A prophylactic or therapeutic agent for spasticity, comprising an RGMa inhibiting substance as an active ingredient.
2. The prophylactic or therapeutic agent according to 1 above, wherein the spasticity is spasticity associated with a disease caused by a spinal cord disorder or a brain disorder.
3. The prophylactic or therapeutic agent according to 1 above, wherein the spasticity is spasticity associated with spinal cord injury.
4. The prophylactic or therapeutic agent according to 1 above, wherein the spasticity is spasticity associated with HTLV-1 associated myelopathy.
5. The agent according to any one of 1 to 4 above, wherein the RGMa inhibiting substance is an anti-RGMa neutralizing antibody.
6. The agent according to 5 above, wherein the anti-RGMa neutralizing antibody is a humanized antibody.
7. The agent according to 5 or 6 above, wherein the anti-RGMa neutralizing antibody is an antibody recognizing an amino acid sequence selected from SEQ ID NO: 16, SEQ ID NO: 36, SEQ ID NO: 37, SEQ ID NO: 38 and SEQ ID NO: 39.
8. The agent according to any one of 5 to 7 above, wherein the anti-RGMa neutralizing antibody is an antibody selected from the following (a) to (I):
   (a) an anti-RGMa neutralizing antibody comprising a light chain variable region comprising LCDR1 comprising the amino acid sequence of SEQ ID NO: 5, LCDR2 comprising the amino acid sequence of SEQ ID NO: 6 and LCDR3 comprising the amino acid sequence of SEQ ID NO: 7, and a heavy chain variable region comprising HCDR1 comprising the amino acid sequence of SEQ ID NO: 8, HCDR2 comprising the amino acid sequence of SEQ ID NO: 9 and HCDR3 comprising the amino acid sequence of SEQ ID NO: 10,
   (b) an anti-RGMa neutralizing antibody comprising a light chain variable region comprising LCDR1 comprising the amino acid sequence of SEQ ID NO: 11, LCDR2 comprising the amino acid sequence of SEQ ID NO: 12 and LCDR3 comprising the amino acid sequence of SEQ ID NO: 13, and a heavy chain variable region comprising HCDR1 comprising the amino acid sequence of SEQ ID NO: 14, HCDR2 comprising the amino acid sequence of SEQ ID NO: 15 and HCDR3 comprising the amino acid sequence of SFG,
   (c) an anti-RGMa neutralizing antibody comprising a light chain variable region comprising LCDR1 comprising the amino acid sequence of SEQ ID NO: 17, LCDR2 comprising the amino acid sequence of SEQ ID NO: 18 and LCDR3 comprising the amino acid sequence of SEQ ID NO: 19, and a heavy chain variable region comprising HCDR1 comprising the amino acid sequence of SEQ ID NO: 20, HCDR2 comprising the amino acid sequence of SEQ ID NO: 21 and HCDR3 comprising the amino acid sequence of SEQ ID NO: 22,
   (d) an anti-RGMa neutralizing antibody comprising a light chain variable region comprising LCDR1 comprising the amino acid sequence of SEQ ID NO: 23, LCDR2 comprising the amino acid sequence of SEQ ID NO: 24 and LCDR3 comprising the amino acid sequence of SEQ ID NO: 25, and a heavy chain variable region comprising HCDR1 comprising the amino acid sequence of SEQ ID NO: 26, HCDR2 comprising the amino acid sequence of SEQ ID NO: 27 and HCDR3 comprising the amino acid sequence of SEQ ID NO: 28,
   (e) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 31, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34,
   (f) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 35, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34,
   (g) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 40, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34,
   (h) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 41, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34,
   (i) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 42, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34,
   (j) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 43, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34,
   (k) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 44, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34, and
   (l) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 45, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34.
9. The agent according to any one of 5 to 8 above, wherein the anti-RGMa neutralizing antibody is the following (a) or (f):
   (a) an anti-RGMa neutralizing antibody comprising a light chain variable region comprising LCDR1 comprising the amino acid sequence of SEQ ID NO: 5, LCDR2 comprising the amino acid sequence of SEQ ID NO: 6 and LCDR3 comprising the amino acid sequence of SEQ ID NO: 7, and a heavy chain variable region comprising HCDR1 comprising the amino acid sequence of SEQ ID NO: 8, HCDR2 comprising the amino acid sequence of SEQ ID NO: 9 and HCDR3 comprising the amino acid sequence of SEQ ID NO: 10,
   (f) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 35, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34.
10. A method for prophylaxis or therapy of spasticity, comprising administering an effective amount of an RGMa inhibiting substance to a mammal in need of treatment.
11. The prophylactic or therapeutic method according to 10 above, wherein the RGMa inhibiting substance is an anti-RGMa neutralizing antibody.
12. Use of an RGMa inhibiting substance in the manufacture of a prophylactic or therapeutic agent for spasticity.
13. The use according to 12 above, wherein the RGMa inhibiting substance is an anti-RGMa neutralizing antibody.
14. A prophylactic or therapeutic agent for spasticity and neurogenic bladder, comprising an RGMa inhibiting substance as an active ingredient.
15. The prophylactic or therapeutic agent according to 14 above, wherein the spasticity and neurogenic bladder are spasticity and neurogenic bladder associated with a disease caused by a spinal cord disorder or a brain disorder.
16. The prophylactic or therapeutic agent according to 14 above, wherein the spasticity and neurogenic bladder are spasticity and neurogenic bladder associated with spinal cord injury.
17. The prophylactic or therapeutic agent according to 14 above, wherein the spasticity and neurogenic bladder are spasticity and neurogenic bladder associated with HTLV-1 associated myelopathy.
18. The agent according to any one of 14 to 17 above, wherein the RGMa inhibiting substance is an anti-RGMa neutralizing antibody.
19. The agent according to 18 above, wherein the anti-RGMa neutralizing antibody is a humanized antibody.
20. The agent according to 18 or 19 above, wherein the anti-RGMa neutralizing antibody is an antibody recognizing an amino acid sequence selected from SEQ ID NO: 16, SEQ ID NO: 36, SEQ ID NO: 37, SEQ ID NO: 38 and SEQ ID NO: 39.
21. The agent according to any one of 18 to 20 above, wherein the anti-RGMa neutralizing antibody is an antibody selected from the following (a) to (I):
   (a) an anti-RGMa neutralizing antibody comprising a light chain variable region comprising LCDR1 comprising the amino acid sequence of SEQ ID NO: 5, LCDR2 comprising the amino acid sequence of SEQ ID NO: 6 and LCDR3 comprising the amino acid sequence of SEQ ID NO: 7, and a heavy chain variable region comprising HCDR1 comprising the amino acid sequence of SEQ ID NO: 8, HCDR2 comprising the amino acid sequence of SEQ ID NO: 9 and HCDR3 comprising the amino acid sequence of SEQ ID NO: 10,
   (b) an anti-RGMa neutralizing antibody comprising a light chain variable region comprising LCDR1 comprising the amino acid sequence of SEQ ID NO: 11, LCDR2 comprising the amino acid sequence of SEQ ID NO: 12 and LCDR3 comprising the amino acid sequence of SEQ ID NO: 13, and a heavy chain variable region comprising HCDR1 comprising the amino acid sequence of SEQ ID NO: 14, HCDR2 comprising the amino acid sequence of SEQ ID NO: 15 and HCDR3 comprising the amino acid sequence of SFG,
   (c) an anti-RGMa neutralizing antibody comprising a light chain variable region comprising LCDR1 comprising the amino acid sequence of SEQ ID NO: 17, LCDR2 comprising the amino acid sequence of SEQ ID NO: 18 and LCDR3 comprising the amino acid sequence of SEQ ID NO: 19, and a heavy chain variable region comprising HCDR1 comprising the amino acid sequence of SEQ ID NO: 20, HCDR2 comprising the amino acid sequence of SEQ ID NO: 21 and HCDR3 comprising the amino acid sequence of SEQ ID NO: 22,
   (d) an anti-RGMa neutralizing antibody comprising a light chain variable region comprising LCDR1 comprising the amino acid sequence of SEQ ID NO: 23, LCDR2 comprising the amino acid sequence of SEQ ID NO: 24 and LCDR3 comprising the amino acid sequence of SEQ ID NO: 25, and a heavy chain variable region comprising HCDR1 comprising the amino acid sequence of SEQ ID NO: 26, HCDR2 comprising the amino acid sequence of SEQ ID NO: 27 and HCDR3 comprising the amino acid sequence of SEQ ID NO: 28,
   (e) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 31, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34,
   (f) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 35, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34,
   (g) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 40, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34,
   (h) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 41, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34,
   (i) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 42, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34,
   (j) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 43, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34,
   (k) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 44, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34, and
   (l) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 45, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34.
22. The agent according to any one of 18 to 21 above, wherein the anti-RGMa neutralizing antibody is the following (a) or (f):
   (a) an anti-RGMa neutralizing antibody comprising a light chain variable region comprising LCDR1 comprising the amino acid sequence of SEQ ID NO: 5, LCDR2 comprising the amino acid sequence of SEQ ID NO: 6 and LCDR3 comprising the amino acid sequence of SEQ ID NO: 7, and a heavy chain variable region comprising HCDR1 comprising the amino acid sequence of SEQ ID NO: 8, HCDR2 comprising the amino acid sequence of SEQ ID NO: 9 and HCDR3 comprising the amino acid sequence of SEQ ID NO: 10,
   (f) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 35, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34.
23. A method for prophylaxis or therapy of spasticity and neurogenic bladder, comprising administering an effective amount of an RGMa inhibiting substance to a mammal in need of treatment.
24. The prophylactic or therapeutic method according to 23 above, wherein the RGMa inhibiting substance is an anti-RGMa neutralizing antibody.
25. Use of an RGMa inhibiting substance in the manufacture of a prophylactic or therapeutic agent for spasticity and neurogenic bladder.
26. The use according to 25 above, wherein the RGMa inhibiting substance is an anti-RGMa neutralizing antibody.
27. A prophylactic or therapeutic agent for neurogenic bladder, comprising an RGMa inhibiting substance as an active ingredient.
28. The prophylactic or therapeutic agent according to 27 above, wherein the neurogenic bladder is neurogenic bladder associated with a disease caused by a spinal cord disorder or a brain disorder.
29. The prophylactic or therapeutic agent according to 27 above, wherein the neurogenic bladder is neurogenic bladder associated with spinal cord injury.
30. The prophylactic or therapeutic agent according to 27 above, wherein the neurogenic bladder is neurogenic bladder associated with HTLV-1 associated myelopathy.
31. The agent according to any one of 27 to 30 above, wherein the RGMa inhibiting substance is an anti-RGMa neutralizing antibody.
32. The agent according to 31 above, wherein the anti-RGMa neutralizing antibody is a humanized antibody.
33. The agent according to any one of 31 or 32 above, wherein the anti-RGMa neutralizing antibody is the following (a):
   (a) an anti-RGMa neutralizing antibody comprising a light chain variable region comprising LCDR1 comprising the amino acid sequence of SEQ ID NO: 5, LCDR2 comprising the amino acid sequence of SEQ ID NO: 6 and LCDR3 comprising the amino acid sequence of SEQ ID NO: 7, and a heavy chain variable region comprising HCDR1 comprising the amino acid sequence of SEQ ID NO: 8, HCDR2 comprising the amino acid sequence of SEQ ID NO: 9 and HCDR3 comprising the amino acid sequence of SEQ ID NO: 10.
34. A method for prophylaxis or therapy of neurogenic bladder, comprising administering an effective amount of an RGMa inhibiting substance to a mammal in need of treatment.
35. The prophylactic or therapeutic method according to 34 above, wherein the RGMa inhibiting substance is an anti-RGMa neutralizing antibody.
36. Use of an RGMa inhibiting substance in the manufacture of a prophylactic or therapeutic agent for neurogenic bladder.
37. The use according to 36 above, wherein the RGMa inhibiting substance is an anti-RGMa neutralizing antibody.

### Advantageous Effects of the Invention

According to the present invention, an RGMa inhibiting substance, particularly an anti-RGMa neutralizing antibody, is useful as a prophylactic or therapeutic agent for associated symptoms of diseases caused by a spinal cord disorder or a brain disorder, for example, injury or inflammation of the spinal cord or brain. Specific examples of the associated symptoms include spasticity or neurogenic bladder and the like.

According to the present invention, an RGMa inhibiting substance, particularly an anti-RGMa neutralizing antibody, is useful as a prophylactic or therapeutic agent for spasticity and/or neurogenic bladder.

### Brief Description of Drawings

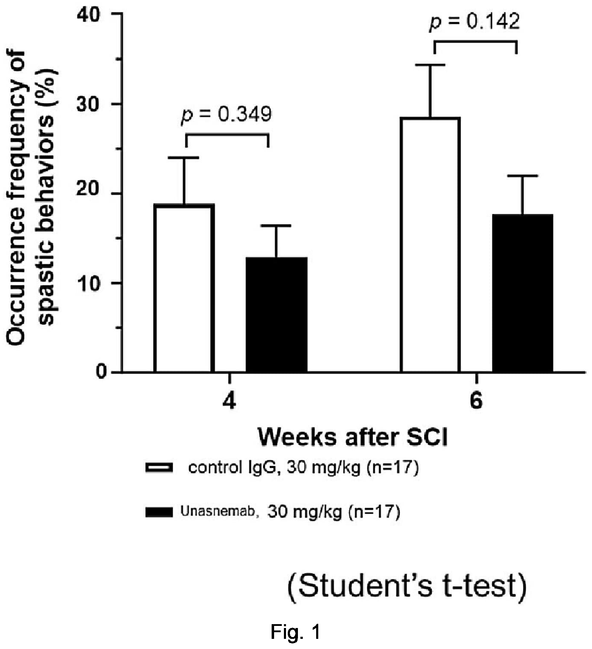

[Figure 1] Figure 1 is a diagram showing the improvement effect of repeated administration of an anti-RGMa neutralizing antibody containing the amino acid sequences of (a) (SEQ ID NOs: 5 to 10) (specifically, Unasnemab) on swimming stress-induced spasticity in a rat spinal cord contusion model, by analysis of the frequency of occurrence of spasticity.

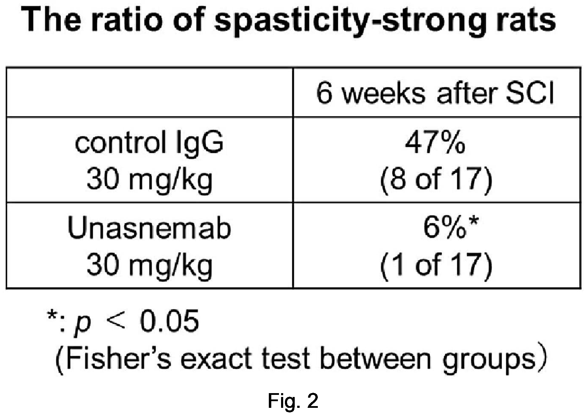

[Figure 2] Figure 2 is a table showing the improvement effect of repeated administration of an anti-RGMa neutralizing antibody containing the amino acid sequences of (a) (SEQ ID NOs: 5 to 10) (specifically, Unasnemab) on swimming stress-induced spasticity in a rat spinal cord contusion model, by analysis of the ratio of individuals with strong expression of spasticity.

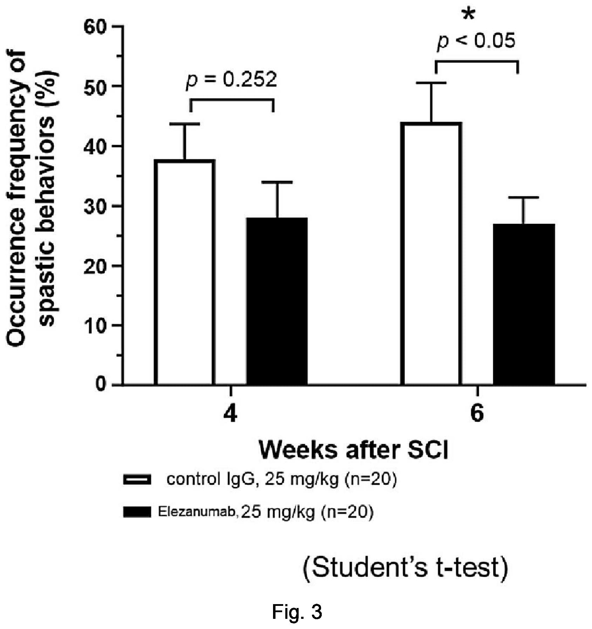

[Figure 3] Figure 3 is a diagram showing the improvement effect of repeated administration of an anti-RGMa neutralizing antibody containing the amino acid sequences of (f) (SEQ ID NOs: 29, 30, 35 and 32 to 34) (specifically, Elezanumab) on swimming stress-induced spasticity in a rat spinal cord contusion model, by analysis of the frequency of occurrence of spasticity.

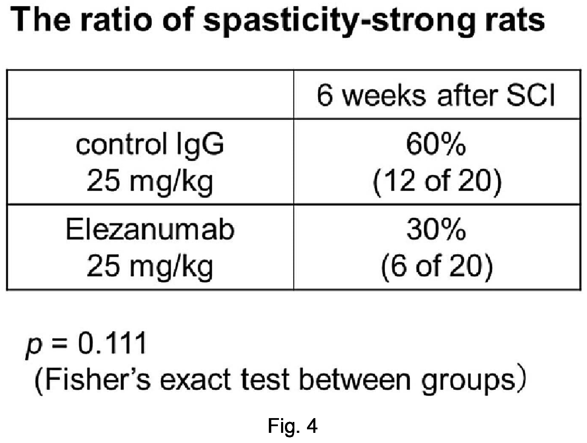

[Figure 4] Figure 4 is a table showing the improvement effect of repeated administration of an anti-RGMa neutralizing antibody containing the amino acid sequences of (f) (SEQ ID NOs: 29, 30, 35 and 32 to 34) (specifically, Elezanumab) on swimming stress-induced spasticity in a rat spinal cord contusion model, by analysis of the ratio of individuals with strong expression of spasticity.

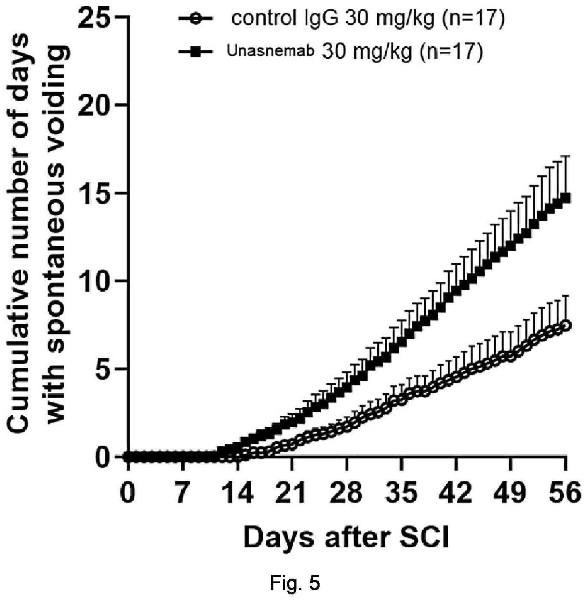

[Figure 5] Figure 5 is a diagram showing the improvement effect of repeated administration of an anti-RGMa neutralizing antibody containing the amino acid sequences of (a) (SEQ ID NOs: 5 to 10) (specifically, Unasnemab) on voiding dysfunction in a rat spinal cord contusion model, by the transition of the cumulative number of days on which spontaneous voiding was performed.

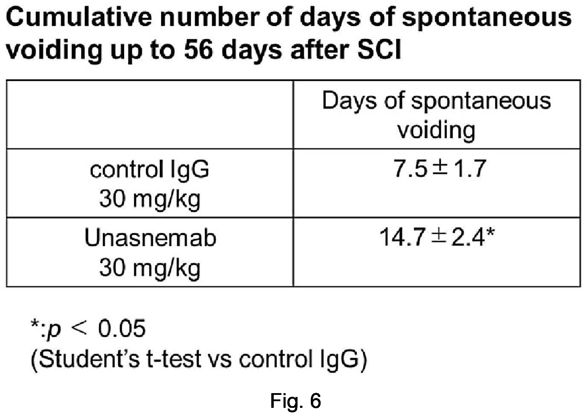

[Figure 6] Figure 6 is a table showing the improvement effect of repeated administration of an anti-RGMa neutralizing antibody containing the amino acid sequences of (a) (SEQ ID NOs: 5 to 10) (specifically, Unasnemab) on voiding dysfunction in a rat spinal cord contusion model, by analysis of the total number of days on which spontaneous voiding was performed.

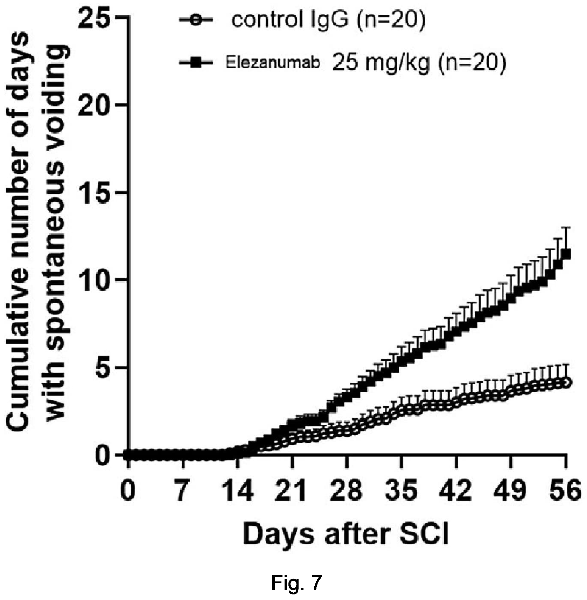

[Figure 7] Figure 7 is a diagram showing the improvement effect of repeated administration of an anti-RGMa neutralizing antibody containing the amino acid sequences of (f) (SEQ ID NOs: 29, 30, 35 and 32 to 34) (specifically, Elezanumab) on voiding dysfunction in a rat spinal cord contusion model, by the transition of the cumulative number of days on which spontaneous voiding was performed.

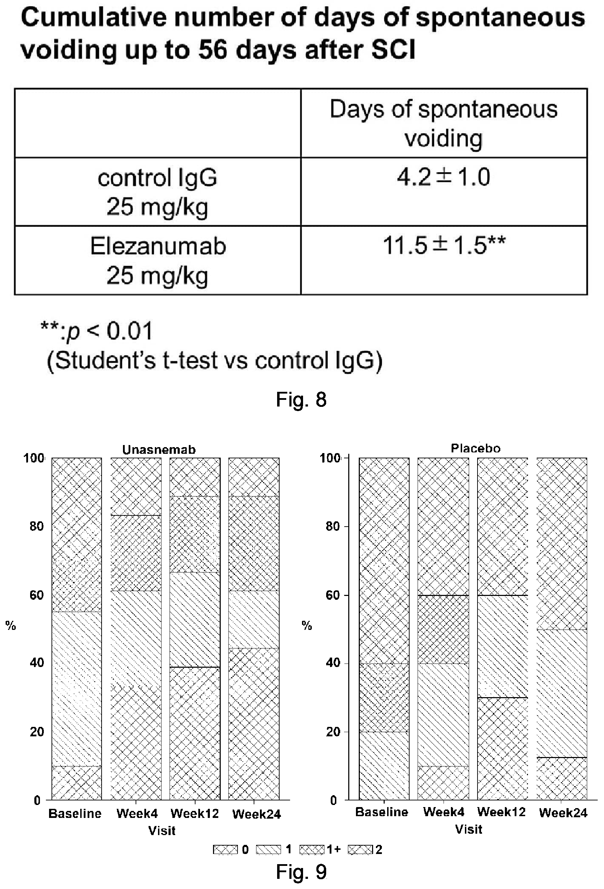

[Figure 8] Figure 8 is a table showing the improvement effect of repeated administration of an anti-RGMa neutralizing antibody containing the amino acid sequences of (f) (SEQ ID NOs: 29, 30, 35 and 32 to 34) (specifically, Elezanumab) on voiding dysfunction in a rat spinal cord contusion model, by analysis of the total number of days on which spontaneous voiding was performed.
[Figure 9] Figure 9 is a graph showing the MAS distribution of both legs (2 limbs) at baseline, 4 weeks, 12 weeks, and 24 weeks in the Unasnemab administration group and the placebo group.

### Description of Embodiments

Hereinafter, terms used in the present invention will be explained.

### <Neutralization>

In the present application, neutralization refers to an action capable of binding to a target of interest and inhibiting any function of that target. For example, an RGMa inhibiting substance refers to a substance that exhibits an action of inhibiting the biological activity of RGMa as a result of binding to RGMa.

### <Epitope>

In the present application, an epitope includes a polypeptide determinant capable of specifically binding to an immunoglobulin or a T cell receptor. In certain embodiments, an epitope comprises chemically active surface group of molecules (e.g., amino acids, sugar side chains, phosphoryl or sulfonyl), and in certain embodiments, may have specific three-dimensional structural characteristics and/or specific charge characteristics. An epitope is a region of an antigen that is bound by an antibody.

### <Isolated>

In the present application, "isolated" regarding an isolated RGMa inhibiting substance (e.g., antibody, etc.) or the like means identified and separated and/or recovered from a component of its natural environment. Contaminant components of its natural environment are materials that would interfere with diagnostic or therapeutic uses for the antibody, and may include enzymes, hormones, and other proteinaceous or non-proteinaceous solutes. Generally, to isolate an RGMa inhibiting substance or the like, it is sufficient to purify it by at least one purification step, and an RGMa inhibiting substance purified by at least one purification step can be referred to as an "isolated RGMa inhibiting substance".

### <Antibody>

In the present application, an antibody broadly refers to an Ig molecule composed of four polypeptide chains, two heavy chains (H chains) and two light chains (L chains), that retains the characteristic of substantially binding to an epitope of an immunoglobulin (Ig) molecule.

### <Human Antibody>

In the present application, a human antibody refers to an antibody in which both the light chain and the heavy chain are derived from human immunoglobulin. Human antibodies include IgG (including IgG1, IgG2, IgG3 and IgG4) having a γ heavy chain, IgM having a µ heavy chain, IgA (including IgA1 and IgA2) having an α heavy chain, IgD having a δ heavy chain, or IgE having an ε heavy chain, depending on the difference in the constant region of the heavy chain. In principle, the light chain comprises either a κ chain or a λ chain.

### <Humanized Antibody>

In the present application, a humanized antibody refers to an antibody composed of a variable region consisting of complementarity determining regions of a non-human animal-derived antibody and framework regions derived from a human antibody, and a constant region derived from a human antibody.

### <Chimeric Antibody>

In the present application, a chimeric antibody refers to an antibody in which the light chain, the heavy chain, or both consist of a variable region derived from a non-human source and a constant region derived from a human source.

### <Monospecific Antibody>

In the present application, a monospecific antibody is an antibody having a single independent antigen recognition site with a single antigen specificity. In the present specification, for example, a monospecific antibody that recognizes RGMa may be referred to as an RGMa monospecific antibody.

### <Multispecific Antibody>

In the present application, a multispecific antibody is an antibody having two or more independent antigen recognition sites with two or more different antigen specificities, and examples thereof include bispecific antibodies having two antigen specificities, trispecific antibodies having three antigen specificities, and the like.

### <Complementarity Determining Region (CDR)>

A complementarity determining region (CDR) refers to a region that forms an antigen-binding site in the variable region of an immunoglobulin molecule, is also called a hypervariable region, and refers to a portion where the variation in amino acid sequence is particularly large for each immunoglobulin molecule. CDRs have three CDRs in each of the light chain and the heavy chain. The three CDRs contained in the light chain may be referred to as LCDR1, LCDR2 and LCDR3, respectively, and the three CDRs contained in the heavy chain may be referred to as HCDR1, HCDR2 and HCDR3. For example, the CDRs of an immunoglobulin molecule are determined according to the Kabat numbering system (Kabat et al., 1987 and 1991, Sequences of Proteins of Immunological Interest, US Department of Health and Human Services, NIH, USA). In the present specification, unless otherwise specified, CDR sequences defined according to the Kabat numbering system are used.

In addition, for the CDR sequences of SEQ ID NOs: 5 to 15, CDR sequences defined according to the numbering system of IMGT (Lefranc M. -P., Immunology Today 18, 509 (1997), Lefranc M. -P., The Immunologist, 7, 132-136 (1999), Lefranc, M.-P., Pommie, C, Ruiz, M., Giudicelli, V., Foulquier, E., Truong, L., Thouvenin-Contet, V. and Lefranc, Dev. Comp. Immunol, 27, 55-77 (2003)) are used. It is obvious to those skilled in the art to replace the CDR sequences described by the IMGT numbering system with any other numbering system, for example, the Kabat numbering system. Therefore, an invention in which the CDR sequences described by the IMGT numbering system are replaced by the Kabat numbering system is included in the embodiments of the present invention. Also, an invention in which the CDR sequences described by the Kabat numbering system are replaced by the IMGT numbering system is also included in the embodiments of the present invention.

### <Effective Amount>

An effective amount refers to an amount of a prophylactic or therapeutic agent sufficient to reduce or improve the severity and/or duration of a disorder or one or more symptoms thereof, prevent the progression of a disorder, regress a disorder, prevent the recurrence, occurrence, onset or progression of one or more symptoms associated with a disorder, detect a disorder, or enhance or improve one or more prophylactic or therapeutic effects of another therapy (e.g., a prophylactic agent or therapeutic agent).

### <Percent (%) Identity of Amino Acid Sequences>

"Percent (%) identity" of an amino acid sequence of a candidate polypeptide sequence, such as a variable region, with respect to an amino acid sequence of a reference polypeptide sequence is defined as the percentage of amino acid residues in a candidate sequence that are identical to the amino acid residues in a specific reference polypeptide sequence, after aligning the sequences and introducing gaps, if necessary, to achieve the maximum % identity, and not considering any conservative substitutions as part of the sequence identity. Alignment for purposes of measuring % identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. However, for purposes herein, % identity values are obtained by using the sequence comparison computer program BLAST in pairwise alignment.

In situations where BLAST is used for amino acid sequence comparisons, the % identity of a given amino acid sequence A to a given amino acid sequence B is calculated as follows:
100 times the fraction X/Y where X is the number of amino acid residues scored as identical matches by the sequence alignment program BLAST in that program's alignment of A and B, and where Y is the total number of amino acid residues in B. It will be appreciated that where the length of amino acid sequence A is not equal to the length of amino acid sequence B, the % identity of A to B will not equal the % identity of B to A. Unless otherwise stated, all % identity values herein are obtained using the BLAST computer program as indicated in the paragraph immediately above.

### <Conservative Substitution>

Conservative substitution means replacing an amino acid residue with another chemically similar amino acid residue so as not to substantially alter the activity of the peptide. Examples include cases where a certain hydrophobic residue is replaced by another hydrophobic residue, cases where a certain polar residue is replaced by another polar residue having the same charge, and the like. Examples of functionally similar amino acids capable of such substitution include, as non-polar (hydrophobic) amino acids, alanine, valine, isoleucine, leucine, proline, tryptophan, phenylalanine, methionine, and the like. Examples of polar (neutral) amino acids include glycine, serine, threonine, tyrosine, glutamine, asparagine, cysteine, and the like. Examples of positively charged (basic) amino acids include arginine, histidine, lysine, and the like. Further, examples of negatively charged (acidic) amino acids include aspartic acid, glutamic acid, and the like.

### <Therapy (Treatment)>

Therapy or treatment includes any treatment of a disease in a subject to be treated, preferably a mammal, particularly a human, and includes arresting the progression of the disease and symptoms, and eliminating, curing, alleviating or mitigating such disease and symptoms.

### < Prophylaxis (Prevention)>

Prophylaxis or prevention includes preventing or suppressing the onset of the above-mentioned disease in a subject to be treated, preferably a mammal, particularly a human. Furthermore, "prophylaxis" in the present invention includes "prophylaxis of recurrence" which prevents the recurrence of the above-mentioned disease that repeatedly remits and recurs in a subject to be treated, preferably a mammal, particularly a human.

Hereinafter, embodiments of the present invention will be described in detail.

The present invention provides a prophylactic or therapeutic agent for associated symptoms of diseases caused by a spinal cord disorder or a brain disorder, for example, diseases caused by injury or inflammation of the spinal cord or brain, which is a novel use of an RGMa inhibiting substance, particularly an anti-RGMa neutralizing antibody.

The present invention also provides a method for prophylaxis or therapy of associated symptoms of diseases caused by a spinal cord disorder or a brain disorder, for example, diseases caused by injury or inflammation of the spinal cord or brain, comprising the step of administering a prophylactic or therapeutic agent comprising an effective amount of an RGMa inhibiting substance, particularly an anti-RGMa neutralizing antibody, to a mammal in need of treatment.

### <RGMa inhibiting substance>

The RGMa inhibiting substance of the present invention may be any substance that acts on RGMa itself to inhibit or attenuate the activity of RGMa (hereinafter, simply referred to as "RGMa activity" in the present specification). For example, a substance having an activity of binding to RGMa and directly inhibiting (attenuating) RGMa activity, or an activity of inhibiting the binding between RGMa and a receptor and indirectly inhibiting (attenuating) RGMa activity (for example, compounds, antibodies, etc. described later) is referred to as the RGMa inhibiting substance of the present invention.

Further, the RGMa inhibiting substance of the present invention may be a substance that suppresses the expression of RGMa; for example, a substance that inhibits the expression of RGMa and inhibits (attenuates) RGMa activity (for example, nucleic acid molecules, etc. described later) is also included in the RGMa inhibiting substance of the present invention.

RGMa was identified as a neurite growth inhibitory protein in the central nervous system, and the human RGMa protein is biosynthesized as a precursor protein consisting of 450 amino acids as shown in SEQ ID NO: 1. The signal peptide Met1-Pro47 (referring to the peptide from the 1st methionine residue to the 47th proline residue from the N-terminal side; hereinafter described similarly) present at the N-terminus is removed, the peptide bond between Asp168 and Pro169 is cleaved to generate an N-terminal domain, and further, the C-terminal peptide Ala425-Cys450 of the fragment on the C-terminal side from Pro169 is removed, and a GPI anchor is added to the C-terminal carboxyl group of Ala424 which has become the C-terminus, generating a C-terminal domain. The human RGMa protein is expressed on the cell membrane via a GPI anchor as a mature protein in which the N-terminal domain (Cys48-Asp168) and the C-terminal domain (Pro169-Ala424) are linked by a disulfide bond.

In the present invention, RGMa may be derived from any animal, but is preferably human RGMa. The precursor protein of human RGMa consists of the amino acid sequence shown in SEQ ID NO: 1 in the Sequence Listing. The precursor protein of mouse RGMa consists of the amino acid sequence shown in SEQ ID NO: 2 in the Sequence Listing, and the precursor protein of rat RGMa consists of the amino acid sequence shown in SEQ ID NO: 3 in the Sequence Listing, but since the C-terminal peptide is removed, they have the same amino acid sequence as the mature protein.

Examples of the RGMa gene include the human RGMa gene consisting of the nucleotide sequence shown in SEQ ID NO: 4, but are not limited thereto. The nucleotide sequences of RGMa genes derived from various organisms can be easily obtained from known databases (GenBank, etc.).

Specific examples of the RGMa inhibiting substance of the present invention include small molecule compounds, anti-RGMa neutralizing antibodies, functionally modified antibodies thereof, conjugated antibodies thereof, or antigen-binding fragments thereof, and also include siRNA (short interfering RNA), shRNA (short hairpin RNA), or antisense oligonucleotides, which are nucleic acid molecules of RGMa. Among these RGMa inhibiting substances, anti-RGMa neutralizing antibodies, functionally modified antibodies thereof, conjugated antibodies thereof, and antigen-binding fragments thereof are preferred, anti-RGMa neutralizing antibodies or antigen-binding fragments thereof are more preferred, and anti-RGMa neutralizing antibodies are particularly preferred.

### <Anti-RGMa Neutralizing Antibody>

In the present invention, the anti-RGMa neutralizing antibody may be any antibody that binds to RGMa and neutralizes RGMa activity, and may be a polyclonal antibody or a monoclonal antibody. In the present invention, a monoclonal antibody is preferred. Further, the anti-RGMa neutralizing antibody of the present invention may be an RGMa monospecific antibody or a multispecific antibody that recognizes RGMa and multiple other antigens, but is preferably an RGMa monospecific antibody.

Further, specific epitopes in human RGMa are preferably one or more selected from SEQ ID NO: 16 (amino acid numbers 47-69 of SEQ ID NO: 1), SEQ ID NO: 36 (amino acid numbers 298-311 of SEQ ID NO: 1), SEQ ID NO: 37 (amino acid numbers 322-335 of SEQ ID NO: 1), SEQ ID NO: 38 (amino acid numbers 349-359 of SEQ ID NO: 1), and SEQ ID NO: 39 (amino acid numbers 367-377 of SEQ ID NO: 1), more preferably a combination of SEQ ID NO: 36 and 37, and particularly preferably a combination of SEQ ID NO: 36, 37 and 39.

The anti-RGMa neutralizing antibody of the present invention includes polyclonal antibodies and monoclonal antibodies obtained by immunizing mammals such as mice with RGMa protein or a partial fragment thereof (e.g., the above-mentioned epitope fragment) as an antigen, chimeric antibodies and humanized antibodies produced using genetic recombination techniques, and human antibodies produced using human antibody-producing transgenic animals and the like. When the antibody of the present invention is administered to humans as a pharmaceutical, a humanized antibody or a human antibody is desirable from the viewpoint of side effects.

Specific examples of the anti-RGMa neutralizing antibody of the present invention include the following antibodies (a) to (I), and for each, the production methods described in Patent Documents 2-4 can be used.
(a) an anti-RGMa neutralizing antibody comprising a light chain variable region comprising LCDR1 comprising the amino acid sequence of SEQ ID NO: 5, LCDR2 comprising the amino acid sequence of SEQ ID NO: 6 and LCDR3 comprising the amino acid sequence of SEQ ID NO: 7, and a heavy chain variable region comprising HCDR1 comprising the amino acid sequence of SEQ ID NO: 8, HCDR2 comprising the amino acid sequence of SEQ ID NO: 9 and HCDR3 comprising the amino acid sequence of SEQ ID NO: 10 (said anti-RGMa neutralizing antibody also includes antibodies whose epitopes are SEQ ID NOs: 36, 37 and 39),
   SEQ ID NO: 5: QDISSY
   SEQ ID NO: 6: YTS (qualifier: mol_type = protein; organism = Mus musculus)
   SEQ ID NO: 7: QQLNTLPWT
   SEQ ID NO: 8: GFTFSDAW
   SEQ ID NO: 9: IRSKANNHAT
   SEQ ID NO: 10: TRRDGAY
(b) an anti-RGMa neutralizing antibody comprising a light chain variable region comprising LCDR1 comprising the amino acid sequence of SEQ ID NO: 11, LCDR2 comprising the amino acid sequence of SEQ ID NO: 12 and LCDR3 comprising the amino acid sequence of SEQ ID NO: 13, and a heavy chain variable region comprising HCDR1 comprising the amino acid sequence of SEQ ID NO: 14, HCDR2 comprising the amino acid sequence of SEQ ID NO: 15 and HCDR3 comprising the amino acid sequence AGS (said anti-RGMa neutralizing antibody also includes antibodies whose epitopes are SEQ ID NOs: 36, 37 and 38),
   SEQ ID NO: 11: QSLVHSNGNTY
   SEQ ID NO: 12: KVS (qualifier: mol_type = protein; organism = Mus musculus)
   SEQ ID NO: 13: SQSTHVPYT
   SEQ ID NO: 14: GYSITTSYY
   SEQ ID NO: 15: ISYDGTN
(c) an anti-RGMa neutralizing antibody comprising a light chain variable region comprising LCDR1 comprising the amino acid sequence of SEQ ID NO: 17, LCDR2 comprising the amino acid sequence of SEQ ID NO: 18 and LCDR3 comprising the amino acid sequence of SEQ ID NO: 19, and a heavy chain variable region comprising HCDR1 comprising the amino acid sequence of SEQ ID NO: 20, HCDR2 comprising the amino acid sequence of SEQ ID NO: 21 and HCDR3 comprising the amino acid sequence of SEQ ID NO: 22,
   SEQ ID NO: 17: RSSQSLEYSDGYTFLE
   SEQ ID NO: 18: EVSNRFS
   SEQ ID NO: 19: FQATHDPLT
   SEQ ID NO: 20: NYGMN
   SEQ ID NO: 21: MIYYDSSEKHYADSVKG
   SEQ ID NO: 22: GTTPDY
(d) an anti-RGMa neutralizing antibody comprising a light chain variable region comprising LCDR1 comprising the amino acid sequence of SEQ ID NO: 23, LCDR2 comprising the amino acid sequence of SEQ ID NO: 24 and LCDR3 comprising the amino acid sequence of SEQ ID NO: 25, and a heavy chain variable region comprising HCDR1 comprising the amino acid sequence of SEQ ID NO: 26, HCDR2 comprising the amino acid sequence of SEQ ID NO: 27 and HCDR3 comprising the amino acid sequence of SEQ ID NO: 28,
   SEQ ID NO: 23: QASQDIDNYLA
   SEQ ID NO: 24: GATNLAD
   SEQ ID NO: 25: LQGYIPPRT
   SEQ ID NO: 26: SYVMH
   SEQ ID NO: 27: YIIPYNDNTKYNEKFKG
   SEQ ID NO: 28: ARRNEYYGSSFFDY
(e) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 31, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34 (said anti-RGMa neutralizing antibody also includes antibodies whose epitope is SEQ ID NO: 16),
   SEQ ID NO: 29: TGTSSSVGDSIYVS
   SEQ ID NO: 30: DVTKRPS
   SEQ ID NO: 31: CSYAGTDTL
   SEQ ID NO: 32: SHGIS
   SEQ ID NO: 33: WISPYSGNTNYAQKLQG
   SEQ ID NO: 34: VGSGPYYYMDV
(f) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 35, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34 (said anti-RGMa neutralizing antibody also includes antibodies whose epitope is SEQ ID NO: 16),
   SEQ ID NO: 29: TGTSSSVGDSIYVS
   SEQ ID NO: 30: DVTKRPS
   SEQ ID NO: 35: YSYAGTDTL
   SEQ ID NO: 32: SHGIS
   SEQ ID NO: 33: WISPYSGNTNYAQKLQG
   SEQ ID NO: 34: VGSGPYYYMDV
(g) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 40, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34 (said anti-RGMa neutralizing antibody also includes antibodies whose epitope is SEQ ID NO: 16),
   SEQ ID NO: 29: TGTSSSVGDSIYVS
   SEQ ID NO: 30: DVTKRPS
   SEQ ID NO: 40: FSYAGTDTL
   SEQ ID NO: 32: SHGIS
   SEQ ID NO: 33: WISPYSGNTNYAQKLQG
   SEQ ID NO: 34: VGSGPYYYMDV
(h) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 41, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34 (said anti-RGMa neutralizing antibody also includes antibodies whose epitope is SEQ ID NO: 16),
   SEQ ID NO: 29: TGTSSSVGDSIYVS
   SEQ ID NO: 30: DVTKRPS
   SEQ ID NO: 41: HSYAGTDTL
   SEQ ID NO: 32: SHGIS
   SEQ ID NO: 33: WISPYSGNTNYAQKLQG
   SEQ ID NO: 34: VGSGPYYYMDV
(i) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 42, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34 (said anti-RGMa neutralizing antibody also includes antibodies whose epitope is SEQ ID NO: 16),
   SEQ ID NO: 29: TGTSSSVGDSIYVS
   SEQ ID NO: 30: DVTKRPS
   SEQ ID NO: 42: LSYAGTDTL
   SEQ ID NO: 32: SHGIS
   SEQ ID NO: 33: WISPYSGNTNYAQKLQG
   SEQ ID NO: 34: VGSGPYYYMDV
(j) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 43, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34 (said anti-RGMa neutralizing antibody also includes antibodies whose epitope is SEQ ID NO: 16),
   SEQ ID NO: 29: TGTSSSVGDSIYVS
   SEQ ID NO: 30: DVTKRPS
   SEQ ID NO: 43: VSYAGTDTL
   SEQ ID NO: 32: SHGIS
   SEQ ID NO: 33: WISPYSGNTNYAQKLQG
   SEQ ID NO: 34: VGSGPYYYMDV
(k) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 44, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34 (said anti-RGMa neutralizing antibody also includes antibodies whose epitope is SEQ ID NO: 16),
   SEQ ID NO: 29: TGTSSSVGDSIYVS
   SEQ ID NO: 30: DVTKRPS
   SEQ ID NO: 44: ISYAGTDTL
   SEQ ID NO: 32: SHGIS
   SEQ ID NO: 33: WISPYSGNTNYAQKLQG
   SEQ ID NO: 34: VGSGPYYYMDV
(l) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 45, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34 (said anti-RGMa neutralizing antibody also includes antibodies whose epitope is SEQ ID NO: 16),
   SEQ ID NO: 29: TGTSSSVGDSIYVS
   SEQ ID NO: 30: DVTKRPS
   SEQ ID NO: 45: KSYAGTDTL
   SEQ ID NO: 32: SHGIS
   SEQ ID NO: 33: WISPYSGNTNYAQKLQG
   SEQ ID NO: 34: VGSGPYYYMDV.

Among these, antibodies described in (a) or (f) are particularly preferred.

Further, examples of the anti-RGMa neutralizing antibody of the present invention include antibodies described in WO 2016/175236, WO 2009/106356, and WO 2013/112922, and the antibodies described in these publications are also included in the anti-RGMa neutralizing antibody of the present invention. Specifically, examples of the anti-RGMa neutralizing antibody of the present invention include antibodies selected from the following (a') to (i').
(a') an anti-RGMa neutralizing antibody comprising a light chain variable region comprising the amino acid sequence of SEQ ID NO: 46 and a heavy chain variable region comprising the amino acid sequence of SEQ ID NO: 47,
   SEQ ID NO: 46: 
DIQMTQSPSSVSASVGDRVTITCRASQDISSYLNWYQQKPGKAPKLLIYYTSRLHS
GVPSRFSGSGSGTDFTLTISSLQPEDFASYFCQQLNTLPWTFGGGTKVEME
   SEQ ID NO: 47: 
EVQLVESGGGLVQPGRSLRLSCTASGFTFSDAWMDWVRQAPGKGLEWVAEIRS
KANNHATYYAESVKGRFTISRDDSKSIVYLQMNSLRTEDTALYYCTRRDGAYWGK
GTTVTVSS
(b') an anti-RGMa neutralizing antibody comprising a light chain comprising the amino acid sequence of SEQ ID NO: 48 and a heavy chain comprising the amino acid sequence of SEQ ID NO: 49 (hereinafter, this antibody is also referred to as Unasnemab),
   SEQ ID NO: 48: 
DIQMTQSPSSVSASVGDRVTITCRASQDISSYLNWYQQKPGKAPKLLIYYTSRLHS
GVPSRFSGSGSGTDFTLTISSLQPEDFASYFCQQLNTLPWTFGGGTKVEMERTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
   SEQ ID NO: 49: 
EVQLVESGGGLVQPGRSLRLSCTASGFTFSDAWMDWVRQAPGKGLEWVAEIRS
KANNHATYYAESVKGRFTISRDDSKSIVYLQMNSLRTEDTALYYCTRRDGAYWGK
GTTVTVSSASTKGPSVFPLAPSSKSTSGGTAALGCLVKDYFPEPVTVSWNSGALT
SGVHTFPAVLQSSGLYSLSSVVTVPSSSLGTQTYICNVNHKPSNTKVDKKVEPKS
CDKTHTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKA
LPAPIEKTISKAKGQPREPQVYTLPPSRDELTKNQVSLTCLVKGFYPSDIAVEWES
 
NGQPENNYKTTPPVLDSDGSFFLYSKLTVDKSRWQQGNVFSCSVMHEALHNHY
TQKSLSLSPGK
(c') an anti-RGMa neutralizing antibody comprising a light chain variable region comprising the amino acid sequence of SEQ ID NO: 50 and a heavy chain variable region comprising the amino acid sequence of SEQ ID NO: 51,
   SEQ ID NO: 50: 
DVVMTQTPLSLPVSLGDQASISCRSSQSLVHSNGNTYLHWYLQRPGQSPKLLIYK
VSNRFSGVPDRFSGSGSGTDFTLKISRVEAEDLGLYFCSQSTHVPYTFGGGTKLE
IK
   SEQ ID NO: 51: 
DVKLQESGPGLVKPSQSLSLTCSVTGYSITTSYYWNWIRQFPGNKLEWMGYISYD
GTNNYNPSLKNRISITRDTSKNQFFLRLNSVTTEDTATYYCAGSFGYSQGTLVTVS
A
(d') an anti-RGMa neutralizing antibody comprising a light chain comprising the amino acid sequence of SEQ ID NO: 52 and a heavy chain comprising the amino acid sequence of SEQ ID NO: 53,
   SEQ ID NO: 52: 
DVVMTQTPLSLPVSLGDQASISCRSSQSLVHSNGNTYLHWYLQRPGQSPKLLIYK
VSNRFSGVPDRFSGSGSGTDFTLKISRVEAEDLGLYFCSQSTHVPYTFGGGTKLE
IKRADAAPTVSIFPPSSEQLTSGGASVVCFLNNFYPKDINVKWKIDGSERQNGVLN
SWTDQDSKDSTYSMSSTLTLTKDEYERHNSYTCEATHKTSTSPIVKSFNRNEC
   SEQ ID NO: 53: 
DVKLQESGPGLVKPSQSLSLTCSVTGYSITTSYYWNWIRQFPGNKLEWMGYISYD
GTNNYNPSLKNRISITRDTSKNQFFLRLNSVTTEDTATYYCAGSFGYSQGTLVTVS
AAKTTPPSVYPLAPGCGDTTGSSVTLGCLVKGYFPESVTVTWNSGSLSSSVHTFP
ALLQSGLYTMSSSVTVPSSTWPSQTVTCSVAHPASSTTVDKKLEPSGPISTINPCP
PCKECHKCPAPNLEGGPSVFIFPPNIKDVLMISLTPKVTCVVVDVSEDDPDVQISW
 
FVNNVEVHTAQTQTHREDYNSTIRVVSTLPIQHQDWMSGKEFKCKVNNKDLPSPI
ERTISKIKGLVRAPQVYILPPPAEQLSRKDVSLTCLVVGFNPGDISVEWTSNGHTEE
NYKDTAPVLDSDGSYFIYSKLNMKTSKWEKTDSFSCNVRHEGLKNYYLKKTISRS
PGK
(e') an anti-RGMa neutralizing antibody comprising a light chain variable region comprising the amino acid sequence of SEQ ID NO: 54 and a heavy chain variable region comprising the amino acid sequence of SEQ ID NO: 55,
   SEQ ID NO: 54: 
DVVLTQTPVSLSVTLGDQASMSCRSSQSLEYSDGYTFLEWFLQKPGQSPQLLIYE
VSNRFSGVPDRFIGSGSGTDFTLKISRVEPEDLGVYYCFQATHDPLTFGSGTKLEI
KR
   SEQ ID NO: 55: 
EVQLVESGGGLVQPGSSLKLSCVASGFTFSNYGMNWIRQAPKKGLEWIGMIYYD
SSEKHYADSVKGRFTISRDNSKNTLYLEMNSLRSEDTAIYYCAKGTTPDYWGQGV
MVTVSS
(f') an anti-RGMa neutralizing antibody comprising a light chain variable region comprising the amino acid sequence of SEQ ID NO: 56 and a heavy chain variable region comprising the amino acid sequence of SEQ ID NO: 57,
   SEQ ID NO: 56: 
DIQMTQSPASLSASLEEIVTITCQASQDIDNYLAWYHQKPGKSPRLLIYGATNLADG
VPSRFSGSRSGTQFSLKINRLQIEDLGIYYCLQGYIPPRTFGGGTKLELKR
   SEQ ID NO: 57: 
EVQLQQSGPELVKPGTSVKMSCKTSGYTFTSYVMHWVKQKPGQGLEWIGYIIPY
NDNTKYNEKFKGKATLTSDKSSSTAYMELSSLTSEDSAVYYCARRNEYYGSSFFD
YWGQGTTLTVSS
(g') an anti-RGMa neutralizing antibody comprising a light chain variable region (lambda chain) comprising the amino acid sequence of SEQ ID NO: 58 and a heavy chain variable region comprising the amino acid sequence of SEQ ID NO: 59,
   SEQ ID NO: 58: 
QSALTQPRSVSGSPGQSVTISCTGTSSSVGDSIYVSWYQQHPGKAPKLMLYDVT
KRPSGVPDRFSGSKSGNTASLTISGLQAEDEADYYCCSYAGTDTLFGGGTKVTVL
   SEQ ID NO: 59: 
EVQLVQSGAEVKKPGASVKVSCKASGYTFTSHGISWVRQAPGQGLDWMGWISP
YSGNTNYAQKLQGRVTMTTDTSTSTAYMELSSLRSEDTAVYYCAR
VGSGPYYYMDVWGQGTLVTVSS
(h') an anti-RGMa neutralizing antibody comprising a light chain variable region comprising the amino acid sequence of SEQ ID NO: 60 and a heavy chain variable region comprising the amino acid sequence of SEQ ID NO: 59, and
   SEQ ID NO: 60: 
QSALTQPRSVSGSPGQSVTISCTGTSSSVGDSIYVSWYQQHPGKAPKLMLYDVT
KRPSGVPDRFSGSKSGNTASLTISGLQAEDEADYYCYSYAGTDTLFGGGTKVTVL
   SEQ ID NO: 59: 
EVQLVQSGAEVKKPGASVKVSCKASGYTFTSHGISWVRQAPGQGLDWMGWISP
YSGNTNYAQKLQGRVTMTTDTSTSTAYMELSSLRSEDTAVYYCAR
VGSGPYYYMDVWGQGTLVTVSS
(i') an anti-RGMa neutralizing antibody comprising a light chain comprising the amino acid sequence of SEQ ID NO: 61 and a heavy chain comprising the amino acid sequence of SEQ ID NO: 62 (hereinafter, this antibody is also referred to as Elezanumab).
   SEQ ID NO: 61: 
QSALTQPRSVSGSPGQSVTISCTGTSSSVGDSIYVSWYQQHPGKAPKLMLYDVT
KRPSGVPDRFSGSKSGNTASLTISGLQAEDEADYYCYSYAGTDTLFGGGTKVTVL
 
GQPKAAPSVTLFPPSSEELQANKATLVCLISDFYPGAVTVAWKADSSPVKAGVET
TTPSKQSNNKYAASSYLSLTPEQWKSHRSYSCQVTHEGSTVEKTVAPTECS
   SEQ ID NO: 62:

EVQLVQSGAEVKKPGASVKVSCKASGYTFTSHGISWVRQAPGQGLDWMGWISP
YSGNTNYAQKLQGRVTMTTDTSTSTAYMELSSLRSEDTAVYYCARVGSGPYYYM
DVWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDKK
VEPKSCDKTHTCPPCPAPEAAGGPSVFLFPPKPKDQLMISRTPEVTCVVVDVSHE
DPEVKFNWYVDGVEVHNAKTKPREEQYNSTYRVVSVLTVLHQDWLNGKEYKCK
VSNKALPAPIEKTISKAKGQPREPQVYTLPPSREEMTKNQVSLTCLVKGFYPSDIA
VEWESNGQPENNYKTTPPVLDSDGSFFLYSKLTVDKSRWQQGNVFSCSVLHEAL
HNHYTQKSLSLSPGK

Existing generally used production methods can be used as the method for producing the anti-RGMa neutralizing antibody of the present invention. The antigen may be used for immunization as it is, or may be used as a complex with a carrier protein. A condensing agent such as glutaraldehyde, carbodiimide, or maleimide active ester can be used for preparing the complex of the antigen and the carrier protein. Examples of carrier proteins include bovine serum albumin, thyroglobulin, hemocyanin, KLH, and the like.

Examples of mammals to be immunized include mice, rats, hamsters, guinea pigs, rabbits, cats, dogs, pigs, goats, horses, cattle, and the like, and inoculation methods include subcutaneous, intramuscular, or intraperitoneal administration. Upon administration, it may be administered mixed with Complete Freund's Adjuvant or Incomplete Freund's Adjuvant, and administration is usually performed once every 2 to 5 weeks. Antibody-producing cells obtained from the spleen or lymph nodes of the immunized animal are fused with myeloma cells to isolate hybridomas. As myeloma cells, those derived from mammals, for example, mice, rats, humans, etc., are used.

### <Polyclonal Antibody>

Polyclonal antibodies can be obtained from serum obtained from an immunized animal by immunizing the above-mentioned mammal with an antigen as described above, together with Freund's Adjuvant if necessary.

### <Monoclonal Antibody>

Specifically, monoclonal antibodies can be obtained as follows. That is, using an antigen as described above as an immunogen, immunization is performed by injecting or transplanting the immunogen into the subcutaneous, intramuscular, intravenous, footpad, or intraperitoneal area of a mammal as described above once to several times, together with Freund's Adjuvant if necessary. Usually, immunization is performed 1 to 4 times every about 1 to 14 days from the first immunization, and antibody-producing cells are obtained from the immunized mammal about 1 to 5 days after the final immunization.

Monoclonal antibodies can be obtained using methods well known to those skilled in the art (e.g., "Current Protocols in Molecular Biology" (John Wiley & Sons (1987)), Antibodies: A Laboratory Manual, Ed. Harlow and David Lane, Cold Spring Harbor Laboratory (1988)).

Preparation of "hybridomas" secreting monoclonal antibodies can be performed according to the method of Kohler and Milstein et al. (Nature, 256, 495, 1975) and modified methods thereof. That is, they are prepared by fusing antibody-producing cells contained in the spleen or the like obtained from an immunized mammal with myeloma cells derived from a mammal, preferably a mouse, rat, or human, which do not have the ability to produce autoantibodies.

Examples of myeloma cells used for cell fusion include mouse-derived myelomas P3/X63-AG8.653 (653), P3/NSI/1-Ag4-1 (NS-1), P3/X63-Ag8.U1 (P3U1), SP2/0-Ag14 (Sp2/O, Sp2), PAI, F0, or BW5147; rat-derived myeloma 210RCY3-Ag.2.3.; and human-derived myelomas U-266AR1, GM1500-6TG-A1-2, UC729-6, CEM-AGR, D1R11, or CEM-T15.

Examples of fusion promoters include polyethylene glycol and the like, and usually, cell fusion can be carried out by reacting for about 1 to 10 minutes at a temperature of 20 to 40°C, preferably 30 to 37°C, using polyethylene glycol (average molecular weight 1000 to 4000) at a concentration of about 20 to 50%, with the ratio of the number of antibody-producing cells to the number of myeloma cells usually being about 1:1 to 10:1.

Screening of hybridoma clones producing monoclonal antibodies can be performed by culturing hybridomas, for example, in microtiter plates, and measuring the reactivity of the culture supernatant of the wells to the immunizing antigen by an immunochemical method such as ELISA.

In the screening of antibody-producing hybridomas, in addition to binding assays with RGMa protein, evaluation is also performed as to whether the antibody inhibits the RGMa activity of the present invention. By these screening methods, the anti-RGMa neutralizing antibody of the present invention can be selected.

From the wells containing hybridomas producing the target antibody, cloning can be further performed by limiting dilution to obtain clones. Selection and breeding of hybridomas are usually performed in a medium for animal cells containing 10 to 20% fetal bovine serum with the addition of HAT (hypoxanthine, aminopterin, thymidine).

Production of monoclonal antibodies from hybridomas can be performed by culturing the hybridomas in vitro, or by growing them in vivo such as in the ascites of mammals such as mice and rats, and isolating from the obtained culture supernatant or mammalian ascites.

In the case of culturing in vitro, it is possible to use a nutrient medium suitable for proliferating, maintaining, and preserving hybridomas and producing monoclonal antibodies in the culture supernatant, in accordance with various conditions such as the characteristics of the cell type to be cultured and the culture method. As the nutrient medium, known nutrient media or nutrient media prepared from basal media can be listed.

Examples of the basal medium include low-calcium media such as Ham's F12 medium, MCDB153 medium, or low-calcium MEM medium, and high-calcium media such as MCDB104 medium, MEM medium, D-MEM medium, RPMI1640 medium, ASF104 medium, or RD medium. The basal medium can contain, for example, serum, hormones, cytokines, and/or various inorganic or organic substances, depending on the purpose.

Isolation and purification of monoclonal antibodies can be performed by subjecting the above-mentioned culture supernatant or ascites to saturated ammonium sulfate, euglobulin precipitation method, caproic acid method, caprylic acid method, ion exchange chromatography (DEAE or DE52, etc.), affinity column chromatography such as anti-immunoglobulin column or protein A column, and the like. Specifically, purification of monoclonal antibodies may be performed using methods known as purification methods for immunoglobulins, and can be easily achieved by means such as ammonium sulfate fractionation, PEG fractionation, ethanol fractionation, use of anion exchangers, and further affinity chromatography using RGMa protein.

Monoclonal antibodies can also be obtained by the phage display method. In the phage display method, phages selected from an arbitrary phage antibody library are screened using a target immunogen to select phages having desired binding properties to the immunogen. Next, the antibody-corresponding sequence contained in the phage is isolated or sequenced, and based on the isolated sequence or the determined sequence information, an expression vector containing a nucleic acid molecule encoding the antibody or antigen-binding domain is constructed. Then, by culturing a cell line transfected with such an expression vector, monoclonal antibodies can be produced. By using a human antibody library as the phage antibody library, human antibodies having desired binding properties can be generated.

### <Nucleic Acid Molecule>

A nucleic acid molecule encoding the anti-RGMa neutralizing antibody or antigen-binding fragment thereof of the present invention can be obtained, for example, by the following method. First, total RNA is prepared from cells such as hybridomas using a commercially available RNA extraction kit, and cDNA is synthesized by reverse transcriptase using random primers or the like. Next, cDNA encoding the antibody is amplified by PCR using oligonucleotides of sequences conserved in the variable regions of known human antibody heavy chain and light chain genes as primers. Sequences encoding constant regions can be obtained by amplifying known sequences by PCR. The nucleotide sequence of the DNA can be determined by a conventional method, such as by incorporating it into a plasmid for sequencing.

Alternatively, DNA encoding the monoclonal antibody of the present invention can be obtained by chemically synthesizing the sequence of the variable region or a part thereof and ligating it to a sequence containing a constant region.

The nucleic acid molecule may encode all of the constant regions and variable regions of the heavy chain and light chain, or may encode only the variable regions of the heavy chain and light chain. The nucleotide sequences of the constant regions of the heavy chain and light chain in the case of encoding all of the constant regions and variable regions are preferably those described in Nucleic Acids Research vol. 14, p1779, 1986, The Journal of Biological Chemistry vol. 257, p1516, 1982, and Cell vol. 22, p197, 1980.

### <Functionally Modified Antibody>

A functionally modified antibody of the anti-RGMa neutralizing antibody is prepared by the following methods. For example, when the present anti-RGMa neutralizing antibody is produced using CHO cells in which the 1,6-fucosyltransferase (FUT8) gene has been disrupted as host cells, an antibody with reduced fucose content in the sugar chain and enhanced cell-killing function is obtained, and when produced using CHO cells into which the FUT8 gene has been introduced as host cells, an antibody with low cell-killing function is obtained (International Publication WO 2005/035586, International Publication WO 2002/31140, International Publication WO 00/61739). Also, complement activation function can be regulated by modifying amino acid residues in the Fc region (US Patent No. 6,737,056, US Patent No. 7,297,775, US Patent No. 7,317,091). Furthermore, by using a mutant of the Fc region with enhanced binding to FcRn, which is one of the Fc receptors, the half-life in blood can be extended (Hashiguchi Shuhei et al., Seikagaku, 2010, Vol. 82(8), p710). These functionally modified antibodies can be produced by genetic engineering. By using a mutant of the Fc region with enhanced binding to FcRn, which is one of the Fc receptors, the half-life in blood can be extended (Hashiguchi Shuhei et al., Seikagaku, 2010, Vol. 82(8), p710). These functionally modified antibodies can be produced by genetic engineering.

### <Conjugated Antibody>

Examples of modified molecules of the anti-RGMa neutralizing antibody of the present invention include conjugated antibodies. Examples of conjugated antibodies include conjugated antibodies in which functional molecules other than the anti-RGMa neutralizing antibody of the present application, such as non-peptide polymers like polyethylene glycol (PEG), radioactive substances, toxins, small molecule compounds, cytokines, growth factors (TGF-β, NGF, Neurotrophin, etc.), albumin, enzymes, and other antibodies, are chemically or genetically engineered to bind to the anti-RGMa neutralizing antibody.

When binding PEG as a functional molecule, PEG having a molecular weight of 2000 to 100000 Da, more preferably 10000 to 50000 Da, can be used without limitation, and it may be linear or branched. PEG can be bound to the N-terminal amino group of the amino acid of the anti-RGMa neutralizing antibody or the like, for example, by using an NHS active group.

When a radioactive substance is used as a functional molecule, ¹³¹I, ¹²⁵I, ⁹⁰Y, ⁶⁴Cu, ⁹⁹Tc, ⁷⁷Lu, ²¹¹At, or the like is used. The radioactive substance can be directly bound to the anti-RGMa neutralizing antibody by the Chloramine-T method or the like.

When a toxin is used as a functional molecule, bacterial toxins (e.g., diphtheria toxin), plant toxins (e.g., ricin), low-molecular-weight toxins (e.g., geldanamycin), maytansinoids, calicheamicin, and the like are used.

When a small molecule compound is used as a functional molecule, examples include daunomycin, doxorubicin, methotrexate, mitomycin, neocarzinostatin, vindesine, and fluorescent dyes such as FITC.

When an enzyme is used as a functional molecule, luciferase (e.g., firefly luciferase and bacterial luciferase; US Patent No. 4,737,456), malate dehydrogenase, urease, peroxidase (e.g., horseradish peroxidase (HRPO)), alkaline phosphatase, β-galactosidase, glucoamylase, lysozyme, saccharide oxidase (e.g., glucose oxidase, galactose oxidase, and glucose-6-phosphate dehydrogenase), heterocyclic oxidase (e.g., uricase and xanthine oxidase, etc.), lactoperoxidase, microperoxidase, and the like are used.

As linkers used when chemically binding toxins, small molecule compounds or enzymes, divalent radicals (e.g., alkylene, arylene, heteroarylene), linkers represented by -(CR₂)ₙO(CR₂)ₙ- (R is any substituent, n is a positive integer), repeating units of alkoxy (e.g., polyethyleneoxy, PEG, polymethyleneoxy, etc.) and alkylamino (e.g., polyethyleneamino, Jeffamine^{™}), and diacid esters and amides (including succinate, succinamide, diglycolate, malonate, caproamide, etc.) can be mentioned. Chemical modification methods for binding functional molecules have already been established in this field (D.J. King., Applications and Engineering of Monoclonal antibodies., 1998 T.J. International Ltd, Monoclonal Antibody-Based Therapy of Cancer., 1998 Marcel Dekker Inc; Chari et al., Cancer Res., 1992 Vol 152: 127; Liu et al., Proc Natl Acad Sci USA., 1996 Vol 93: 8681).

### <Antigen-Binding Fragment>

In an embodiment of the present invention, an "antigen-binding fragment" of an antibody means a partial region of an antibody as described above having antigen-binding properties, and specific examples include F(ab')₂, Fab', Fab, Fv (variable fragment of antibody), disulfide-linked Fv, single-chain antibody (scFv), and polymers thereof. Furthermore, antigen-binding fragments include conjugated fragments in which functional molecules other than the anti-RGMa neutralizing antibody of the present application, such as non-peptide polymers like polyethylene glycol (PEG), radioactive substances, toxins, small molecule compounds, cytokines, growth factors (TGF-β, NGF, Neurotrophin, etc.), albumin, enzymes, and other antibodies, are chemically or genetically engineered to bind.

"F(ab')₂" and "Fab" refer to antibody fragments produced by treating immunoglobulin with a proteolytic enzyme such as pepsin or papain, and being digested before or after the disulfide bonds existing between the two heavy chains in the hinge region. For example, when IgG is treated with papain, it is cleaved upstream of the disulfide bonds existing between the two heavy chains in the hinge region to produce two homologous antibody fragments in which a light chain consisting of VL (light chain variable region) and CL (light chain constant region) and a heavy chain fragment consisting of VH (heavy chain variable region) and CHγ1 region (γ1 region in the heavy chain constant region) are linked by a disulfide bond at the C-terminal region. Each of these two homologous antibody fragments is called Fab. Also, when IgG is treated with pepsin, it is cleaved downstream of the disulfide bonds existing between the two heavy chains in the hinge region to produce an antibody fragment slightly larger than one in which the two Fabs are connected at the hinge region. This antibody fragment is called F(ab')₂.

### <Chimeric Antibody>

A preferred embodiment of the anti-RGMa neutralizing antibody of the present invention includes a chimeric antibody. Examples of the "chimeric antibody" include chimeric antibodies in which the variable region is a variable region derived from an immunoglobulin of a non-human animal (mouse, rat, hamster, chicken, etc.) and the constant region is a constant region derived from a human immunoglobulin. For example, it can be prepared by immunizing a mouse with an antigen, excising a variable region that binds to the antigen from the mouse monoclonal antibody gene, and binding it to an antibody constant region derived from human bone marrow. The constant region derived from human immunoglobulin has a unique amino acid sequence depending on the isotype such as IgG (IgG1, IgG2, IgG3, IgG4), IgM, IgA (IgA1, IgA2), IgD, and IgE, but the constant region of the recombinant chimeric antibody in the present invention may be a constant region of human immunoglobulin belonging to any isotype. Preferably, it is a constant region of human IgG. An expression vector can be prepared using the gene of the chimeric antibody prepared in this way. By transforming host cells with the expression vector, chimeric antibody-producing transformed cells are obtained, and by culturing the transformed cells, the target chimeric antibody is obtained from the culture supernatant.

### <Humanized Antibody>

Another preferred embodiment of the anti-RGMa neutralizing antibody of the present invention includes a humanized antibody. The "humanized antibody" in the present invention is an antibody in which only the DNA sequence of the antigen-binding site (CDR; complementarity determining region) of a non-human animal antibody such as a mouse is grafted onto a human antibody gene (CDR grafting). For example, it can be produced with reference to the methods described in JP-T-4-506458, Japanese Patent No. 2912618, etc. Specifically, it means a humanized antibody characterized in that a part or all of its CDRs are CDRs derived from a monoclonal antibody of a non-human mammal (mouse, rat, hamster, etc.), the framework regions of its variable regions are framework regions of variable regions derived from human immunoglobulin, and its constant regions are constant regions derived from human immunoglobulin.

The humanized antibody in the present invention can be produced, for example, as follows. However, it goes without saying that it is not limited to such a production method.

For example, a recombinant humanized antibody derived from a mouse monoclonal antibody can be produced by genetic engineering with reference to JP-T-4-506458 and JP-A-62-296890. That is, DNA of the mouse heavy chain CDR portion and DNA of the mouse light chain CDR portion are isolated from a hybridoma producing a mouse monoclonal antibody, and a human heavy chain gene of the entire region other than the human heavy chain CDR and a human light chain gene of the entire region other than the human light chain CDR are isolated from human immunoglobulin genes.

The human heavy chain gene into which the isolated DNA of the mouse heavy chain CDR portion has been grafted is introduced into an appropriate expression vector so that it can be expressed, and similarly, the human light chain gene into which the DNA of the mouse light chain CDR portion has been grafted is introduced into another appropriate expression vector so that it can be expressed. Alternatively, the human heavy chain and light chain genes into which mouse CDRs have been grafted can be introduced into the same expression vector so that they can be expressed. By transforming host cells with the expression vector prepared in this way, humanized antibody-producing transformed cells are obtained, and by culturing the transformed cells, the target humanized antibody is obtained from the culture supernatant.

### <Human Antibody>

Another preferred embodiment of the anti-RGMa neutralizing antibody of the present invention includes a human antibody. A human antibody is an antibody in which all regions including the variable region of the heavy chain and the constant region of the heavy chain and the variable region of the light chain and the constant region of the light chain constituting the immunoglobulin are derived from genes encoding human immunoglobulin, and can be produced by introducing human antibody genes into mice. Specifically, for example, it can be produced in the same manner as the method for producing polyclonal antibodies or monoclonal antibodies described above by immunizing a transgenic animal prepared by incorporating at least a human immunoglobulin gene into the locus of a mammal other than a human such as a mouse with an antigen.

For example, transgenic mice producing human antibodies can be produced according to the methods described in Nature Genetics, Vol. 7, p. 13-21, 1994; Nature Genetics, Vol. 15, p. 146-156, 1997; JP-T-4-504365; JP-T-7-509137; International Publication WO 94/25585 pamphlet; Nature, Vol. 368, p. 856-859, 1994; and JP-T-6-500233. More specifically, HuMab (registered trademark) mouse (Medarex, Princeton NJ), KM^{™} mouse (Kirin Pharma Company, Japan), KM (FCγRIIb-KO) mouse, and the like can be mentioned.

Specific examples of the anti-RGMa neutralizing antibody of the present invention include those having CDRs containing specific amino acid sequences in the heavy chain variable region and CDRs containing specific amino acid sequences in the light chain variable region (preferably, the anti-RGMa neutralizing antibodies of (a) to (I) described above and (a') to (i') described above).

In addition, as long as the characteristic of the antibody of the present invention of having binding ability to RGMa and inhibiting (neutralizing) RGMa activity is maintained, there may be substitution, deletion, addition or insertion of one or several amino acids (1 to 20, 1 to 10 or 1 to 5, preferably 1 to 2) in the amino acid sequence of the anti-RGMa neutralizing antibody (preferably, the anti-RGMa neutralizing antibodies of (a) to (I) described above and (a') to (i') described above). Such substitution, deletion, addition may be introduced into the CDRs, but is preferably introduced into regions other than the CDRs. Also, the amino acid substitution is preferably a conservative substitution in order to maintain the characteristics of the present invention.

The amino acid sequence of the anti-RGMa neutralizing antibody of the present invention containing substitution, deletion, etc. in the amino acid sequence (preferably, the anti-RGMa neutralizing antibodies of (a) to (I) described above and (a') to (i') described above) is, for example, an amino acid sequence in which the heavy chain variable region after amino acid sequence modification has a % identity of 90% or more (more preferably 95%, 96%, 97%, 98%, 99% or more) with the amino acid sequence before modification, and an amino acid sequence in which the light chain variable region after amino acid sequence modification has a % identity of 90% or more (more preferably 95%, 96%, 97%, 98%, 99% or more) with the amino acid sequence before modification.

In the present invention, siRNA is a short double-stranded RNA capable of suppressing the expression of a target gene (RGMa gene in the present invention). As long as it functions as an siRNA that inhibits the RGMa activity of the present invention, the base sequence and length (base length) are not particularly limited, but are preferably less than about 30 bases, more preferably about 19 to 27 bases, and still more preferably about 21 to 25 bases.

In the present invention, shRNA refers to a molecule of about 20 base pairs or more consisting of a short hairpin structure having a protruding portion at the 3' end by forming a double-stranded structure within the molecule by including a partially palindromic base sequence in a single-stranded RNA. Such shRNA is degraded into a length of about 20 bases (typically, for example, 21 bases, 22 bases, 23 bases) within the cell after being introduced into the cell, and can suppress the expression of the target gene similarly to siRNA.

In the present invention, the above-mentioned siRNA and shRNA may be in any form as long as they can suppress the expression of the RGMa gene.

In the present invention, siRNA or shRNA can be artificially chemically synthesized. Further, for example, antisense and sense RNAs can be synthesized in vitro from template DNA using T7 RNA polymerase and T7 promoter. The antisense oligonucleotide may be any nucleotide that is complementary to or hybridizes with a consecutive sequence of 5 to 100 bases in the DNA sequence of the RGMa gene, and may be either DNA or RNA. Further, it may be modified as long as there is no hindrance to its function. The antisense oligonucleotide can be synthesized by a conventional method, and for example, can be easily synthesized by a commercially available DNA synthesizer.

Preferred sequences can be selected using ordinary selection methods, and the siRNA or shRNA in the present invention can be confirmed by evaluating the expression inhibition of functional RGMa.

### <Prophylactic or Therapeutic Agent for Spasticity and/or Neurogenic Bladder>

In an embodiment of the present invention, spasticity is a disorder of sensorimotor control resulting from an intermittent or sustained involuntary activation of muscles due to an upper motor neuron lesion (Disabil Rehabil. 2005 Jan;27(1-2):2-6).

As for the cause of spasticity, upper motor neurons, which are long nerves extending from the brain to the spinal cord, govern voluntary movements, and when damage or disorder occurs in these neurons, abnormalities arise in the content of messages to the muscles. Spasticity is an abnormal action of reflexes caused by a disruption of the complex balance between nerve excitation and inhibition in the brain or spinal cord due to damage or disorder of the central nervous system that controls voluntary movements. For example, when signals from the brain to the lower part of the body are interrupted by spinal cord injury or spinal cord diseases (compression, trauma, blood flow disturbance, neurodegeneration, etc.), unnecessary muscle contractions, i.e., spasticity, occur due to heightened nerve excitation. Symptoms of spasticity include muscle hypertonia, rapid muscle contraction (spasm), hyperreflexia of deep tendon reflexes, muscle convulsion (clonus), scissoring posture (a state where the hip joints are adducted and internally rotated), joint stiffness, and the like (Pract Neurol. 2012;12(5):289-98).

The present invention includes a prophylactic or therapeutic agent for spasticity and its symptoms, which is a novel use of an RGMa inhibiting substance, particularly an anti-RGMa neutralizing antibody (preferably, an anti-RGMa neutralizing antibody comprising the amino acid sequences of (a) (SEQ ID NOs: 5 to 10) or the amino acid sequences of (f) (SEQ ID NOs: 29, 30, 35 and 32 to 34), and more preferably, an anti-RGMa neutralizing antibody comprising the amino acid sequences of (a') (SEQ ID NOs: 46 and 47), the amino acid sequences of (b') (SEQ ID NOs: 48 and 49), the amino acid sequences of (h') (SEQ ID NOs: 60 and 59), or the amino acid sequences of (i') (SEQ ID NOs: 61 and 62)).

Further, in an embodiment of the present invention, neurogenic bladder is a state in which an abnormality has occurred in lower urinary tract (bladder/urethra) function caused by damage or disorder of the central nervous system, autonomic nervous system, or peripheral nervous system (Neurogenic Bladder and Neurogenic Lower Urinary Tract Dysfunction. In: StatPearls [Internet]. Treasure Island (FL): StatPearls Publishing; 2023 Jan. 2022 Nov 28).

As for the cause of neurogenic bladder, since urination is controlled by coordination and reflexes of the spinal cord, brainstem, and spinal cord, various neurological diseases present with lower urinary tract dysfunction (Transl Androl Urol. 2016 Feb; 5(1): 12-21). It is a particularly common complication in spinal cord injury. This is because the micturition reflex center is located in the sacral cord at the end of the spinal cord, so regardless of which part of the spinal cord is damaged, the neural pathway between the sacral cord involved in urination and the cerebrum is blocked (Management of the neurogenic bladder for adults with spinal cord injuries. 2013). Lower urinary tract dysfunction, which is a symptom of neurogenic bladder, is classified into two types: storage dysfunction and voiding dysfunction (Neurogenic Bladder and Neurogenic Lower Urinary Tract Dysfunction. In: StatPearls [Internet]. Treasure Island (FL): StatPearls Publishing; 2023 Jan. 2022 Nov 28). In storage dysfunction, the bladder contracts arbitrarily, and symptoms such as pollakiuria, urinary urgency, and urinary incontinence appear. In voiding dysfunction, bladder contraction is impaired, and symptoms of dysuria appear.

The present invention includes a prophylactic or therapeutic agent for neurogenic bladder, which is a novel use of an RGMa inhibiting substance, particularly an anti-RGMa neutralizing antibody (preferably, an anti-RGMa neutralizing antibody comprising the amino acid sequences of (a) (SEQ **ID** NOs: 5 to 10) or the amino acid sequences of (f) (SEQ **ID** NOs: 29, 30, 35 and 32 to 34), and more preferably, an anti-RGMa neutralizing antibody comprising the amino acid sequences of (a') (SEQ **ID** NOs: 46 and 47), the amino acid sequences of (b') (SEQ **ID** NOs: 48 and 49), the amino acid sequences of (h') (SEQ **ID** NOs: 60 and 59), or the amino acid sequences of (i') (SEQ ID NOs: 61 and 62)). Further, a prophylactic or therapeutic agent for neurogenic bladder or lower urinary tract dysfunction of an RGMa inhibiting substance, particularly an anti-RGMa neutralizing antibody (preferably, an anti-RGMa neutralizing antibody comprising the amino acid sequences of (a) (SEQ ID NOs: 5 to 10) or the amino acid sequences of (f) (SEQ ID NOs: 29, 30, 35 and 32 to 34), and more preferably, an anti-RGMa neutralizing antibody comprising the amino acid sequences of (a') (SEQ ID NOs: 46 and 47), the amino acid sequences of (b') (SEQ ID NOs: 48 and 49), the amino acid sequences of (h') (SEQ ID NOs: 60 and 59), or the amino acid sequences of (i') (SEQ ID NOs: 61 and 62)) is included.

Furthermore, the present invention also includes a prophylactic or therapeutic agent for spasticity and neurogenic bladder (or lower urinary tract dysfunction) of an RGMa inhibiting substance, particularly an anti-RGMa neutralizing antibody (preferably, an anti-RGMa neutralizing antibody comprising the amino acid sequences of (a) (SEQ ID NOs: 5 to 10) or the amino acid sequences of (f) (SEQ ID NOs: 29, 30, 35 and 32 to 34), and more preferably, an anti-RGMa neutralizing antibody comprising the amino acid sequences of (a') (SEQ ID NOs: 46 and 47), the amino acid sequences of (b') (SEQ ID NOs: 48 and 49), the amino acid sequences of (h') (SEQ ID NOs: 60 and 59), or the amino acid sequences of (i') (SEQ ID NOs: 61 and 62)).

### <Prophylactic or Therapeutic Agent for Associated Symptoms of Diseases Caused by Spinal Cord or Brain Disorders>

In an embodiment of the present invention, the prophylactic or therapeutic agent for associated symptoms of diseases caused by spinal cord or brain disorders, which is a novel use of an RGMa inhibiting substance, means an agent or pharmaceutical composition capable of prophylaxis or therapy of associated symptoms of diseases caused by a spinal cord disorder or a brain disorder, specifically, spinal cord or brain injury or inflammation, for example, spasticity and/or neurogenic bladder (or lower urinary tract dysfunction).
Here, examples of the diseases causing spasticity include cerebral palsy, multiple sclerosis, amyotrophic lateral sclerosis, stroke (including cerebral infarction, cerebral hemorrhage, etc.), brain injury, spinal cord compression, spinal cord injury, motor neuron disease, spinal vascular malformation, hereditary spastic paraplegia (or hereditary spastic paraparesis), subacute combined degeneration of the spinal cord, HTLV-1 associated myelopathy (also known as: tropical spastic paraparesis or tropical spastic paraplegia), copper deficiency, syringomyelia, and tethered cord syndrome (Crit Rev Phys Rehabil Med. 2013;25(1-2):11-22, Pract Neurol. 2012; 12(5):289-98).

Further, examples of the diseases causing neurogenic bladder (or lower urinary tract dysfunction) include spinal/spinal cord diseases or brain diseases. Specifically, examples of spinal/spinal cord diseases include vertebral diseases (herniated disc, spinal canal stenosis, ossification of posterior longitudinal ligament), vascular diseases (spinal cord hemorrhage/infarction), spinal cord tumors, demyelinating diseases (multiple sclerosis, neuromyelitis optica), infectious diseases (myelitis, HTLV-1 associated myelopathy), inflammatory diseases (sarcoidosis, collagen disease), congenital diseases (spina bifida, sacral agenesis, tethered cord syndrome), degenerative diseases (amyotrophic lateral sclerosis), exogenous diseases (spinal cord injury, radiation-induced), hereditary spastic paraplegia, and the like. Further, examples of brain diseases include vascular diseases (cerebral hemorrhage/infarction), brain tumors, degenerative diseases (Parkinson's disease, multiple system atrophy, Alzheimer's disease, spinocerebellar degeneration, olivopontocerebellar atrophy, amyotrophic lateral sclerosis), demyelinating diseases (multiple sclerosis), infectious diseases (encephalitis), inflammatory diseases (sarcoidosis, collagen disease), traumatic diseases (head injury), hereditary spastic paraplegia, and the like (Transl Androl Urol. 2016 Feb; 5(1): 12-21, etc.).

The present invention includes a prophylactic or therapeutic agent for associated symptoms of diseases caused by spinal cord or brain disorders, which is a novel use of an RGMa inhibiting substance, particularly an anti-RGMa neutralizing antibody (preferably, an anti-RGMa neutralizing antibody comprising the amino acid sequences of (a) (SEQ ID NOs: 5 to 10) or the amino acid sequences of (f) (SEQ ID NOs: 29, 30, 35 and 32 to 34), and more preferably, an anti-RGMa neutralizing antibody comprising the amino acid sequences of (a') (SEQ ID NOs: 46 and 47), the amino acid sequences of (b') (SEQ ID NOs: 48 and 49), the amino acid sequences of (h') (SEQ ID NOs: 60 and 59), or the amino acid sequences of (i') (SEQ ID NOs: 61 and 62)). Further, the associated symptoms include spasticity, neurogenic bladder (or lower urinary tract dysfunction), and the like. The present invention includes prophylactic or therapeutic agents for each of the associated symptoms, and also includes prophylactic or therapeutic agents for two associated symptoms (spasticity and neurogenic bladder (or lower urinary tract dysfunction)).

### <Pharmaceutical Composition>

The prophylactic or therapeutic agent for associated symptoms of diseases caused by spinal cord or brain disorders comprising an RGMa inhibiting substance, or the prophylactic or therapeutic agent for spasticity and/or neurogenic bladder comprising an RGMa inhibiting substance in the present invention is usually administered systemically or locally in an oral or parenteral form.

The prophylactic or therapeutic agent for associated symptoms of diseases caused by spinal cord or brain disorders comprising an RGMa inhibiting substance, or the prophylactic or therapeutic agent for spasticity and/or neurogenic bladder comprising an RGMa inhibiting substance in the present invention can be formulated by utilizing an RGMa inhibiting substance as an active ingredient and appropriately blending pharmaceutically acceptable carriers or additives. The pharmaceutical composition thus formulated can be administered in an oral or parenteral form. Specifically, it can be made into oral agents such as tablets, coated tablets, pills, powders, granules, capsules, liquids, suspensions, emulsions, etc., and can also be made into parenteral agents such as injections, infusions, suppositories, ointments, patches, etc. The blending ratio of carriers or additives may be appropriately set based on the range usually adopted in the pharmaceutical field. Carriers or additives that can be blended are not particularly limited, but examples include various carriers such as water, physiological saline, other aqueous solvents, aqueous or oily bases, and various additives such as excipients, binders, pH adjusters, disintegrants, absorption enhancers, lubricants, coloring agents, flavoring agents, and fragrances.

When the RGMa inhibiting substance is an anti-RGMa neutralizing antibody, a function-modified antibody thereof, a conjugate antibody thereof, or an antigen-binding fragment thereof, it is preferably administered via a parenteral administration route, for example, intravenous, intramuscular, intradermal, intraperitoneal, subcutaneous, or local administration, as an injection or infusion formulated with a pharmaceutically acceptable carrier.

For example, an injection or infusion containing an anti-RGMa neutralizing antibody can be used as a solution, suspension, or emulsion. As the solvent, for example, distilled water for injection, physiological saline, glucose solution, and isotonic solution (for example, solutions of sodium chloride, potassium chloride, glycerin, mannitol, sorbitol, boric acid, borax, propylene glycol, etc.) and the like can be used.

Furthermore, such an injection or infusion containing an anti-RGMa neutralizing antibody may contain stabilizers, solubilizers, suspending agents, emulsifiers, soothing agents, buffering agents, preservatives, antiseptics, pH adjusters, and the like.

As stabilizers, for example, albumin, globulin, gelatin, mannitol, glucose, dextran, ethylene glycol, propylene glycol, ascorbic acid, sodium bisulfite, sodium thiosulfate, sodium EDTA, sodium citrate, dibutyl hydroxytoluene, and the like can be used.

As solubilizers, for example, alcohols (e.g., ethanol, etc.), polyalcohols (e.g., propylene glycol, polyethylene glycol, etc.), nonionic surfactants (e.g., Polysorbate 80 (registered trademark), HCO-50, etc.) and the like can be used.

As suspending agents, for example, glyceryl monostearate, aluminum monostearate, methyl cellulose, carboxymethyl cellulose, hydroxymethyl cellulose, sodium lauryl sulfate, and the like can be used.

As emulsifiers, for example, gum arabic, sodium alginate, tragacanth, and the like can be used.

As soothing agents, for example, benzyl alcohol, chlorobutanol, sorbitol, and the like can be used.

As buffering agents, for example, phosphate buffer, acetate buffer, borate buffer, carbonate buffer, citrate buffer, Tris buffer, and the like can be used.

As preservatives, for example, methyl paraoxybenzoate, ethyl paraoxybenzoate, propyl paraoxybenzoate, butyl paraoxybenzoate, chlorobutanol, benzyl alcohol, benzalkonium chloride, sodium dehydroacetate, sodium edetate, boric acid, borax, and the like can be used.

As antiseptics, for example, benzalkonium chloride, paraoxybenzoic acid, chlorobutanol, and the like can be used.

As pH adjusters, for example, hydrochloric acid, sodium hydroxide, phosphoric acid, acetic acid, and the like can be used.

When the RGMa inhibiting substance is a nucleic acid (siRNA, shRNA, antisense oligonucleotide, etc.), it can be administered in the form of a non-viral vector or a viral vector. In the case of a non-viral vector form, methods of introducing nucleic acid molecules using liposomes (liposome method, HVJ-liposome method, cationic liposome method, lipofection method, lipofectamine method, etc.), microinjection methods, methods of transferring nucleic acid molecules into cells together with a carrier (metal particles) using a gene gun, and the like can be utilized. For example, when administering siRNA or shRNA to a living body using a viral vector, viral vectors such as recombinant adenovirus and retrovirus can be utilized. By introducing DNA expressing siRNA or shRNA into DNA viruses or RNA viruses such as detoxified retrovirus, adenovirus, adeno-associated virus, herpes virus, vaccinia virus, pox virus, polio virus, Sindbis virus, Sendai virus, SV40, etc., and infecting cells or tissues with this recombinant virus, the gene can be introduced into the cells or tissues.

The preparation thus obtained can prevent or treat associated symptoms of diseases caused by spinal cord or brain disorders, and can prevent or treat spasticity and/or neurogenic bladder, by administering an effective amount thereof to, for example, humans and other mammals (e.g., rats, mice, rabbits, sheep, pigs, cows, cats, dogs, monkeys, etc.). The dosage is appropriately set in consideration of the purpose, severity of the disease, age, weight, sex, medical history of the patient, type of active ingredient, and the like. For example, when the active ingredient is an anti-RGMa neutralizing antibody, in the case of targeting an average human having a body weight of about 65 to 70 kg, about 0.02 mg to 4000 mg per day is preferable. The total daily dosage may be a single dose or divided doses.

### Examples

The present invention will be described more specifically below with reference to examples, but these do not limit the scope of the present invention. A rat spinal cord contusion model was used as a pathological model of spinal cord injury.

As the anti-RGMa neutralizing antibody, an anti-RGMa neutralizing antibody comprising the amino acid sequences of (a) (SEQ ID NOs: 5 to 10) and the amino acid sequences of (f) (SEQ ID NOs: 29, 30, 35 and 32 to 34) described in the present specification was used, and Palivizumab was used as a control antibody in each example.

### Example 1

### Examination of the Effect of Anti-RGMa Neutralizing Antibody on Spasticity and Dysuria in Rat Spinal Cord Contusion Model

### (1-1) Preparation of Rat Spinal Cord Contusion Model

SD strain female rats (11 weeks old at the time of model preparation) were used for the experiment. The animals were anesthetized with a three-drug mixed anesthetic of medetomidine hydrochloride, midazolam and butorphanol tartrate, and after shaving from the neck to the back, they were wiped with disinfection ethanol and Isodine. During the procedure, isoflurane was inhaled to maintain the anesthetic state. The dorsal skin was incised to expose the 6th thoracic vertebra to the 2nd lumbar vertebra, and then the vertebral arches of the 9th to 10th thoracic vertebrae were removed. Immediately, using a MASCIS Impactor (Rutgers university, USA), a weight of 10 g with a diameter of 2.5 mm was dropped from a height of 50 mm to damage the spinal cord. From the day of spinal cord injury, cefazolin sodium for infection prevention and physiological saline for dehydration prevention were each subcutaneously administered to the buttocks for 7 days.

In principle, from the day after spinal cord injury until the end of the test day, forced urination by manual bladder compression was performed twice a day (morning and afternoon). For individuals showing signs of urinary tract infection in urine color tone during forced urination on and after 8 days after spinal cord injury following the end of the cefazolin sodium administration period, enrofloxacin was subcutaneously administered daily until the urine color tone became normal.

### (1-2) Evaluation of Spasticity

According to the method of Ryu et al. (PLos One 2017;12:e0171937), the frequency of spasticity occurrence was evaluated using a swimming test method in which spastic behaviors occurring in the hind limbs and trunk during swimming are observed. For the swimming test, a rectangular acrylic water tank (width 13 cm, length 150 cm, height 40 cm) was used, and it was carried out under conditions of a water depth of 20 to 23 cm and a water temperature of 20 to 23°C. Observation of behavior until swimming a linear distance of 100 cm from the water entry point to the end point was defined as one trial, and a trial in which spastic behavior such as clonus or spasm was observed even once in the hind limbs or trunk was defined as a spasticity-positive trial. Ten trials were performed per animal at each time point, and the frequency (%) of spasticity-positive trials was calculated. All trials of the swimming test were videotaped, and two observers independently evaluated the spastic behavior in a blinded state so that they did not know which test substance was administered to which individual. The average of the spasticity occurrence frequency (%) calculated by each observer was taken as the representative value for that evaluation time point. Also, referring to the paper by Ryu et al. (PLos One 2017;12:e0171937), individuals showing a representative value of spasticity occurrence frequency of 35% or more were determined as spasticity-strong expression individuals.

### (1-3) Evaluation of Spontaneous Voiding Ability

Based on the records of the presence or absence of bladder tension and the presence or absence of urination during forced urination every morning, the spontaneous voiding ability was evaluated. It was determined that spontaneous voiding had occurred when there was no bladder tension and urination was not observed even by compression. The evaluation of spontaneous voiding was carried out until 56 days after the preparation of the rat spinal cord contusion model.

### (1-4) Grouping and Administration of Anti-RGMa Neutralizing Antibody

Before the spinal cord contusion treatment, the animals were distributed so that the average body weight of each group was uniform.

As anti-RGMa neutralizing antibodies, an anti-RGMa neutralizing antibody comprising the amino acid sequences of (a) (SEQ ID NOs: 5 to 10) (specifically, Unasnemab) and an anti-RGMa neutralizing antibody comprising the amino acid sequences of (f) (SEQ ID NOs: 29, 30, 35 and 32 to 34) (specifically, Elezanumab), which were prepared by the methods described in International Publication WO 2016/175236 and International Publication WO 2013/112922 respectively, were used. The anti-RGMa neutralizing antibodies were prepared so that they could be administered at 3 ml/kg, and were administered as a bolus into the tail vein under isoflurane anesthesia. The initial administration was performed immediately after spinal cord contusion, and thereafter, administration was performed once a week for a total of 8 times. In the examination of Unasnemab, specifically, Unasnemab (30 mg/kg per individual) or a control antibody (Palivizumab 30 mg/kg per individual) was administered. In the examination of Elezanumab, Elezanumab (25 mg/kg per individual) or a control antibody (Palivizumab 25 mg/kg per individual) was administered.

### (1-5) Results

### Effect on Spasticity

The effect of repeated administration of Unasnemab on swimming-induced spasticity in the rat spinal cord contusion model is shown in FIG. 1 by analysis of the frequency of spasticity occurrence. At 4 weeks and 6 weeks after spinal cord injury, the spasticity occurrence frequency in the Unasnemab 30 mg/kg administration group showed a lower value compared to the control antibody administration group.

The effect of repeated administration of Unasnemab on swimming-induced spasticity in the rat spinal cord contusion model is shown in FIG. 2 by analysis of the ratio of spasticity-strong expression individuals. At 6 weeks after spinal cord injury, the ratio of spasticity-strong expression individuals in the Unasnemab 30 mg/kg administration group was significantly decreased compared to the control antibody administration group (Fisher's exact test).

The effect of repeated administration of Elezanumab on swimming-induced spasticity in the rat spinal cord contusion model is shown in FIG. 3 by analysis of the frequency of spasticity occurrence. At 4 weeks and 6 weeks after spinal cord injury, the spasticity occurrence frequency in the Elezanumab 25 mg/kg administration group showed a lower value compared to the control antibody administration group, and at 6 weeks after spinal cord injury, it significantly decreased (Student's t-test).

The effect of repeated administration of Elezanumab on swimming-induced spasticity in the rat spinal cord contusion model is shown in FIG. 4 by analysis of the ratio of spasticity-strong expression individuals. At 6 weeks after spinal cord injury, the ratio of spasticity-strong expression individuals in the Elezanumab 25 mg/kg administration group showed a lower value compared to the control antibody administration group.

### Effect on Dysuria

The effect of repeated administration of Unasnemab on dysuria in the rat spinal cord contusion model is shown in FIG. 5 as the transition of the cumulative number of days on which spontaneous voiding was performed. The cumulative number of days in the Unasnemab (30 mg/kg) administration group remained higher than that in the control antibody administration group.

The effect of repeated administration of Unasnemab on dysuria in the rat spinal cord contusion model is shown in FIG. 6 as an analysis of the total number of days on which spontaneous voiding was performed. The Unasnemab administration group showed a dose-dependent increase in the number of days of spontaneous voiding, and the number of days of spontaneous voiding in the Unasnemab 30 mg/kg administration group significantly increased compared to the control antibody administration group (Student's t-test).

The effect of repeated administration of Elezanumab on dysuria in the rat spinal cord contusion model is shown in FIG. 7 as the transition of the cumulative number of days on which spontaneous voiding was performed. The cumulative number of days in the Elezanumab 25 mg/kg administration group remained higher than that in the control antibody administration group.

The effect of repeated administration of Elezanumab on dysuria in the rat spinal cord contusion model is shown in FIG. 8 as an analysis of the total number of days on which spontaneous voiding was performed. The number of days of spontaneous voiding in the Elezanumab 25 mg/kg administration group significantly increased compared to the control antibody administration group (Student's t-test).

### Example 2

This clinical trial is a Phase Ib, randomized, double-blind, placebo-controlled comparative study with the primary objective of investigating the safety, tolerability, and pharmacokinetics when the active drug, an anti-RGMa neutralizing antibody comprising the amino acid sequences of (a) (SEQ ID NOs: 5 to 10) (specifically, Unasnemab) or placebo, is administered for 20 weeks to patients with HTLV-1 associated myelopathy (hereinafter, HAM) having moderate to high disease activity. In addition, the efficacy of Unasnemab was exploratorily investigated using the following indicators.
(1) Modified Ashworth Scale (hereinafter, MAS) (knee extensors and flexors)
(2) Overactive Bladder Symptom Score (hereinafter, OABSS)

The study was conducted in 15 participants (10 in the active drug group, 5 in the placebo group). Participants deemed eligible to proceed to the treatment period were randomly assigned to the active drug group or the placebo group at a ratio of 2:1. Using neopterin concentration in the cerebrospinal fluid (hereinafter, CSF) at screening as a stratification factor, participants were divided into participants with moderate disease activity (6 to 43 pmol/mL) or participants with high disease activity (44 pmol/mL or more) and stratified assignment was performed.

The active drug or placebo was administered by intravenous infusion over 1 hour or more on the treatment period start day, and at weeks 2, 4, 8, 12, 16, and 20.

The clinical trial period was the period from the day of informed consent to the last day of the follow-up period. The screening period was the period from the day of informed consent onwards until the start of investigational drug administration on the first day of the treatment period (day of first administration of the investigational drug), and was set to a maximum of 6 weeks. The treatment period was the period from the start of investigational drug administration on the first day of the treatment period (day of first administration of the investigational drug) until the last day of the treatment period (24 weeks of the treatment period or the day of discontinuation during the treatment period). The follow-up period was the period from the day following the last day of the treatment period (24 weeks of the treatment period or the day of discontinuation during the treatment period) until 16 weeks after the last administration of the investigational drug.

### (1) MAS (knee extensors and flexors)

MAS is an evaluation index for muscle tone, and was evaluated in 6-point scale of 0, 1, 1+, 2, 3, and 4 (Kansai Rigaku. 2012;12:1-6, Phys ther. 1987;67(2):206-7). MAS shown in Table 1 was evaluated for knee extensors and flexors on the first day of the treatment period (pre-dose) (baseline), week 4, week 12, and week 24.

**Table 1**

| Score | Description |
|---|---|
| 0 | No increase in muscle tone |
| 1 | A feeling of catching occurs during movement and then disappears, or slight resistance is observed at the end of the extension range. |
| 1+ | Muscle tone is slightly increased, and slight resistance is observed after a feeling of catching in a range of less than 1/2 of the range of motion. |
| 2 | Muscle tone is increased throughout the range of motion, but passive movement is easily possible. |
| 3 | Muscle tone is further increased, and passive movement is difficult. |
| 4 | The limbs are rigid and passive movement is impossible. |

### (2) OABSS

OABSS is an evaluation index used for the evaluation of overactive bladder for voiding dysfunction, and the state of the past week was evaluated. The frequency of each of the 4 question items in Table 2 was scored, and the total was evaluated on a scale of 0 to 15 points. In addition, the severity was evaluated in 3 stages: mild (0-5), moderate (6-11), and severe (12-15).

Participants answered the OABSS questionnaire (UROLOGY 68(2): 318-323, 2006) on the first day of the treatment period (pre-dose) (baseline), week 4, week 12, week 24, and week 12 of the follow-up period, and the results were evaluated.

**Table 2**

| Q# | Symptom | Score | Frequency |
|---|---|---|---|
| 1 | How many times did you urinate from the time you woke up in the morning until you went to sleep? | 0 | ≤ 7 times |
| | | 1 | 8-14 times |
| | | 2 | ≥ 15 times |
| 2 | How many times did you wake up to urinate from the time you went to sleep at night until you woke up in the morning? | 0 | 0 times |
| | | 1 | 1 time |
| | | 2 | 2 times |
| | | 3 | ≥ 3 times |
| 3 | Did you have a sudden desire to urinate that was difficult to hold? | 0 | None |
| | | 1 | Less than once a week |
| | | 2 | Once a week or more |
| | | 3 | About once a day |
| | | 4 | 2-4 times a day |
| | | 5 | 5 times a day or more |
| 4 | Did you leak urine because you had a sudden desire to urinate and could not hold it? | 0 | None |
| | | 1 | Less than once a week |
| | | 2 | Once a week or more |
| | | 3 | About once a day |
| | | 4 | 2-4 times a day |
| | | 5 | 5 times a day or more |

### Results

### (1) MAS

The distribution of MAS for both legs (2 limbs) at each evaluation time point is shown in FIG. 9.

The percentage of improvement in MAS for both legs (2 limbs) at 24 weeks of the treatment period was 55.6% (10/18 limbs) in the active drug group and 37.5% (3/8 limbs) in the placebo group, indicating a trend of improvement for spasticity in the active drug group.

### (2) OABSS

The OABSS at each evaluation time point and the change from baseline are shown in Table 3-1 and Table 3-2, and the shift table of severity is shown in Table 4.

The LS Mean ± SE of the change from baseline in OABSS at 24 weeks of the treatment period was -1.6 ± 0.5 in the active drug group and 1.6 ± 0.8 in the placebo group. In the active drug group, the improvement from baseline was greater compared to the placebo group, indicating a trend of improvement for neurogenic bladder.

The percentage of subjects whose severity improved at 24 weeks of the treatment period was 22.2% (2/9 subjects) in the active drug group and 0% (0/4 subjects) in the placebo group. Subjects who worsened were 25.0% (1/4 subjects) in the placebo group, and none were observed in the active drug group (0/9 subjects).

**Table 3-1**

| Analysis visit | Statistics | Placebo (N=5) | Unasnemab (N=10/9) |
|---|---|---|---|
| Baseline | n | 5 | 10 |
| | Mean | 7.0 | 4.5 |
| | SD | 3.2 | 2.6 |
| | Median | 7.0 | 4.0 |
| | (Min, Max) | (3, 11) | (1, 10) |
| Week 4 | n | 5 | 9 |
| | Mean | 6.4 | 3.7 |
| | SD | 2.9 | 2.3 |
| | Median | 7.0 | 3.0 |
| | (Min, Max) | (3, 10) | (0, 7) |
| Change from baseline at Week 4 | n | 5 | 9 |
| | Mean | -0.6 | -1.0 |
| | SD | 2.9 | 1.3 |
| | Median | 0.0 | -1.0 |
| | (Min, Max) | (-5, 3) | (-3, 1) |
| | LS mean (SE) | 0.1(0.8) | -1.4(0.6) |
| | LSMD vs Placebo (SE) | | -1.5(1.0) |
| | 95% Cl | | (-3.7, 0.7) |
| | p-value (a) | | 0.170 |

| | | | |
|---|---|---|---|
| **(a) p-value from MMRM model** | | | |

**Table 3-2**

| Analysis Visit | Statistics | Placebo (N=5) | Unasnemab (N=10) |
|---|---|---|---|
| Week 12 | n | 5 | 9 |
| | Mean | 7.8 | 3.0 |
| | SD | 3.6 | 1.7 |
| | Median | 8.0 | 3.0 |
| | (Min, Max) | (3, 13) | (1, 6) |
| Change from baseline at Week 12 | n | 5 | 9 |
| | Mean | 0.8 | -1.7 |
| | SD | 1.3 | 2.0 |
| | Median | 1.0 | -1.0 |
| | (Min, Max) | (-1, 2) | (-5, 1) |
| | LS mean (SE) | 1.5 (0.7) | -2.1 (0.6) |
| | LSMD vs Placebo | | -3.5 (0.9) |
| | (SE) | | |
| | 95% CI | | (-5.6, -1.5) |
| | p-value (a) | | 0.003 |
| Week 24 | n | 4 | 9 |
| | Mean | 8.5 | 3.4 |
| | SD | 1.3 | 1.8 |
| | Median | 8.5 | 4.0 |
| | (Min, Max) | (7, 10) | (1, 6) |
| Change from baseline at Week 24 | n | 4 | 9 |
| | Mean | 0.5 | -1.2 |
| | SD | 1.3 | 2.2 |
| | Median | 0.5 | 0.0 |
| | (Min, Max) | (-1, 2) | (-5, 1) |
| | LS mean (SE) | 1.6 (0.8) | -1.6 (0.5) |
| | LSMD vs Placebo (SE) | | -3.3 (0.9) |
| | 95% CI | | (-5.3, -1.3) |
| | p-value (a) | | 0.004 |

| | | | |
|---|---|---|---|
| **(a) p-value from MMRM model** | | | |

**Table 4**

| Analysis Visit | Baseline | Placebo(N=5) | | | Unasnemab(N=10) | | |
|---|---|---|---|---|---|---|---|
| | | Mild n(%) | Moderate n(%) | Severe n(%) | Mild n(%) | Moderate n(%) | Severe n(%) |
| Baseline | | 2 (40.0) | 3 (60.0) | 0 | 7 (70.0) | 3 (30.0) | 0 |
| Week 4 | n | | 5 | | | 9 | |
| | Mild | 1 (20.0) | 1 (20.0) | 0 | 6 (66.7) | 1 (11.1) | 0 |
| | Moderate | 1 (20.0) | 2 (40.0) | 0 | 0 | 2 (22.2) | 0 |
| | Severe | 0 | 0 | 0 | 0 | 0 | 0 |
| Week 12 | n | | 5 | | | 9 | |
| | Mild | 1 (20.0) | 0 | 0 | 6 (66.7) | 2 (22.2) | 0 |
| | Moderate | 1 (20.0) | 2 (40.0) | 0 | 0 | 1 (11.1) | 0 |
| | Severe | 0 | 1 (20.0) | 0 | 0 | 0 | 0 |
| Week 24 | n | | 4 | | | 9 | |
| | Mild | 0 | 0 | 0 | 6 (66.7) | 2 (22.2) | 0 |
| | Moderate | 1 (25.0) | 3 (75.0) | 0 | 0 | 1 (11.1) | 0 |
| | Severe | 0 | 0 | 0 | 0 | 0 | 0 |
| FU Week 12 | n | | 5 | | | 10 | |
| | Mild | 1 (20.0) | 0 | 0 | 7 (70.0) | 1 (10.0) | 0 |
| | Moderate | 1 (20.0) | 3 (60.0) | 0 | 0 | 2 (20.0) | 0 |
| | Severe | 0 | 0 | 0 | 0 | 0 | 0 |

| | | | | | | | |
|---|---|---|---|---|---|---|---|
| **Percentages were calculated using the number of subjects with values at baseline and a specific visit as the denominator.** | | | | | | | |

A subgroup analysis was performed on the participant population whose baseline HAM-BDSG (an evaluation index for the severity of voiding dysfunction in HAM) was Grade 0 (no treatment and no lower urinary tract symptoms) or Grade 1 (presence of lower urinary tract symptoms or receiving drug treatment), that is, the population excluding participants performing intermittent catheterization. The OABSS at each evaluation time point and the change from baseline are shown in Table 5-1 and Table 5-2, and the shift table of severity is shown in Table 6.

The mean ± SD of the change from baseline in OABSS at 24 weeks of the treatment period was -1.4 ± 2.3 in the active drug group and 0.5 ± 0.7 in the placebo group. Also in the above subgroup, the improvement from baseline was greater in the active drug group compared to the placebo group, indicating a trend of improvement for neurogenic bladder.

The percentage of participants whose severity improved at 24 weeks of the treatment period was 25.0% (2/8 subjects) in the active drug group and 0% (0/2 subjects) in the placebo group, and no worsened subjects were observed in either group.

**Table 5-1 BDSG: Grade 0 or Grade 1**

| Analysis Visit | Statistics | Placebo (N=3) | Unasnemab (N=9) |
|---|---|---|---|
| Baseline | n | 3 | 9 |
| | Mean | 6.3 | 4.9 |
| | SD | 3.1 | 2.5 |
| | Median | 7.0 | 4.0 |
| | (Min, Max) | (3, 9) | (2, 10) |
| Week 4 | n | 3 | 8 |
| | Mean | 4.7 | 4.1 |
| | SD | 2.1 | 2.0 |
| | Median | 4.0 | 4.0 |
| | (Min, Max) | (3, 7) | (1, 7) |
| Change from baseline at Week 4 | n | 3 | 8 |
| | Mean | -1.7 | -1.0 |
| | SD | 2.9 | 1.4 |
| | Median | 0.0 | -1.0 |
| | (Min, Max) | (-5, 0) | ( -3, 1) |
| Week 12 | n | 3 | 8 |
| | Mean | 6.3 | 3.3 |
| | SD | 2.9 | 1.7 |
| | Median | 8.0 | 3.5 |
| | (Min, Max) | (3, 8) | (1, 6) |
| Change from baseline at Week 12 | n | 3 | 8 |
| | Mean | 0.0 | -1.9 |
| | SD | 1.0 | 2.0 |
| | Median | 0.0 | -1.5 |
| | (Min, Max) | (-1, 1) | ( -5, 1) |

**Table 5-2 BDSG: Grade 0 or Grade 1**

| Analysis Visit | Statistics | Placebo (N=3) | Unasnemab (N=9) |
|---|---|---|---|
| | n | 2 | 8 |
| | Mean | 8.5 | 3.8 |
| Week 24 | SD | 0.7 | 1.7 |
| | Median | 8.5 | 4.0 |
| | (Min, Max) | (8, 9) | (1, 6) |
| | n | 2 | 8 |
| Change from baseline at Week 24 | Mean | 0.5 | -1.4 |
| | SD | 0.7 | 2.3 |
| | Median | 0.5 | -0.5 |
| | (Min, Max) | (0, 1) | (-5, 1) |

**Table 6 BDSG: Grade 0 or Grade 1**

| Analysis Visit | Baseline | Placebo (N=3) | | Unasnemab (N=9) | | | |
|---|---|---|---|---|---|---|---|
| | | Mild n (%) | Moderate (%) n | Severe n (%) | Mild n (%) | Moderate n (%) | Severe n (%) |
| Baseline | | 1 (33.3) | 2 (66.7) | 0 | 6 (66.7) | 3 (33.3) | 0 |
| Week 4 | n | | 3 | | | 8 | |
| | Mild | 1 (33.3) | 1 (33.3) | 0 | 5 (62.5) | 1 (12.5) | 0 |
| | Moderate | 0 | 1 (33.3) | 0 | 0 | 2 (25.0) | 0 |
| | Severe | 0 | 0 | 0 | 0 | 0 | 0 |
| Week 12 | n | | 3 | | | 8 | |
| | Mild | 1 (33.3) | 0 | 0 | 5 (62.5) | 2 (25.0) | 0 |
| | Moderate | 0 | 2 (66.7) | 0 | 0 | 1 (12.5) | 0 |
| | Severe | 0 | 0 | 0 | 0 | 0 | 0 |
| Week 24 | n | | 2 | | | 8 | |
| | Mild | 0 | 0 | 0 | 5 (62.5) | 2 (25.0) | 0 |
| | Moderate | 0 | 2 (100) | 0 | 0 | 1 (12.5) | 0 |
| | Severe | 0 | 0 | 0 | 0 | 0 | 0 |
| FU Week 12 | | | | | | | |
| | n | | 3 | | | 9 | |
| | Mild | 1 (33.3) | 0 | 0 | 6 (66.7) | 1 (11.1) | 0 |
| | Moderate | 0 | 2 (66.7) | 0 | 0 | 2 (22.2) | 0 |
| | Severe | 0 | 0 | 0 | 0 | 0 | 0 |

| | | | | | | | |
|---|---|---|---|---|---|---|---|
| Percentages were calculated using the number of subjects with values at baseline and a specific visit as the denominator. | | | | | | | |

### <Description of Sequence Listing>

SEQ ID NO: 1: Amino acid sequence of human RGMa precursor protein
SEQ ID NO: 2: Amino acid sequence of mouse RGMa precursor protein
SEQ ID NO: 3: Amino acid sequence of rat RGMa precursor protein
SEQ ID NO: 4: DNA sequence of human RGMa gene
SEQ ID NO: 5: Amino acid sequence of LCDR1 of anti-RGMa neutralizing antibody r116A3
SEQ ID NO: 6: Amino acid sequence of LCDR2 of anti-RGMa neutralizing antibody r116A3
SEQ ID NO: 7: Amino acid sequence of LCDR3 of anti-RGMa neutralizing antibody r116A3
SEQ ID NO: 8: Amino acid sequence of HCDR1 of anti-RGMa neutralizing antibody r116A3
SEQ ID NO: 9: Amino acid sequence of HCDR2 of anti-RGMa neutralizing antibody r116A3
SEQ ID NO: 10: Amino acid sequence of HCDR3 of anti-RGMa neutralizing antibody r116A3
SEQ ID NO: 11: Amino acid sequence of LCDR1 of anti-RGMa neutralizing antibody r70E4
SEQ ID NO: 12: Amino acid sequence of LCDR2 of anti-RGMa neutralizing antibody r70E4
SEQ ID NO: 13: Amino acid sequence of LCDR3 of anti-RGMa neutralizing antibody r70E4
SEQ ID NO: 14: Amino acid sequence of HCDR1 of anti-RGMa neutralizing antibody r70E4
SEQ ID NO: 15: Amino acid sequence of HCDR2 of anti-RGMa neutralizing antibody r70E4
SEQ ID NO: 16: Amino acid sequence of epitope of human RGMa
SEQ ID NO: 17: Amino acid sequence of LCDR1 of anti-RGMa neutralizing antibody 5F9
SEQ ID NO: 18: Amino acid sequence of LCDR2 of anti-RGMa neutralizing antibody 5F9
SEQ ID NO: 19: Amino acid sequence of LCDR3 of anti-RGMa neutralizing antibody 5F9
SEQ ID NO: 20: Amino acid sequence of HCDR1 of anti-RGMa neutralizing antibody 5F9
SEQ ID NO: 21: Amino acid sequence of HCDR2 of anti-RGMa neutralizing antibody 5F9
SEQ ID NO: 22: Amino acid sequence of HCDR3 of anti-RGMa neutralizing antibody 5F9
SEQ ID NO: 23: Amino acid sequence of LCDR1 of anti-RGMa neutralizing antibody 8D1
SEQ ID NO: 24: Amino acid sequence of LCDR2 of anti-RGMa neutralizing antibody 8D1
SEQ ID NO: 25: Amino acid sequence of LCDR3 of anti-RGMa neutralizing antibody 8D1
SEQ ID NO: 26: Amino acid sequence of HCDR1 of anti-RGMa neutralizing antibody 8D1
SEQ ID NO: 27: Amino acid sequence of HCDR2 of anti-RGMa neutralizing antibody 8D1
SEQ ID NO: 28: Amino acid sequence of HCDR3 of anti-RGMa neutralizing antibody 8D1
SEQ ID NO: 29: Amino acid sequence of LCDR1 of anti-RGMa neutralizing antibody AE12-1
SEQ ID NO: 30: Amino acid sequence of LCDR2 of anti-RGMa neutralizing antibody AE12-1
SEQ ID NO: 31: Amino acid sequence of LCDR3 of anti-RGMa neutralizing antibody AE12-1
SEQ ID NO: 32: Amino acid sequence of HCDR1 of anti-RGMa neutralizing antibody AE12-1
SEQ ID NO: 33: Amino acid sequence of HCDR2 of anti-RGMa neutralizing antibody AE12-1
SEQ ID NO: 34: Amino acid sequence of HCDR3 of anti-RGMa neutralizing antibody AE12-1
SEQ ID NO: 35: Amino acid sequence of LCDR3 of anti-RGMa neutralizing antibody AE12-1Y
SEQ ID NO: 36: Amino acid sequence of epitope of human RGMa
SEQ ID NO: 37: Amino acid sequence of epitope of human RGMa
SEQ ID NO: 38: Amino acid sequence of epitope of human RGMa
SEQ ID NO: 39: Amino acid sequence of epitope of human RGMa
SEQ ID NO: 40: Amino acid sequence of LCDR3 of anti-RGMa neutralizing antibody AE12-1F
SEQ ID NO: 41: Amino acid sequence of LCDR3 of anti-RGMa neutralizing antibody AE12-1H
SEQ ID NO: 42: Amino acid sequence of LCDR3 of anti-RGMa neutralizing antibody AE12-1L
SEQ ID NO: 43: Amino acid sequence of LCDR3 of anti-RGMa neutralizing antibody AE12-1V
SEQ ID NO: 44: Amino acid sequence of LCDR3 of anti-RGMa neutralizing antibody AE12-1I
SEQ ID NO: 45: Amino acid sequence of LCDR3 of anti-RGMa neutralizing antibody AE12-1K
SEQ ID NO: 46: Amino acid sequence of light chain variable region of anti-RGMa neutralizing antibody r116A3 (humanized antibody) (Unasnemab)
SEQ ID NO: 47: Amino acid sequence of heavy chain variable region of anti-RGMa neutralizing antibody r116A3 (humanized antibody) (Unasnemab)
SEQ ID NO: 48: Amino acid sequence of light chain of anti-RGMa neutralizing antibody r116A3 (humanized antibody) (Unasnemab)
SEQ ID NO: 49: Amino acid sequence of heavy chain of anti-RGMa neutralizing antibody r116A3 (humanized antibody) (Unasnemab)
SEQ ID NO: 50: Amino acid sequence of light chain variable region of anti-RGMa neutralizing antibody r70E4
SEQ ID NO: 51: Amino acid sequence of heavy chain variable region of anti-RGMa neutralizing antibody r70E4
SEQ ID NO: 52: Amino acid sequence of light chain of anti-RGMa neutralizing antibody r70E4
SEQ ID NO: 53: Amino acid sequence of heavy chain of anti-RGMa neutralizing antibody r70E4
SEQ ID NO: 54: Amino acid sequence of light chain variable region of anti-RGMa neutralizing antibody 5F9
SEQ ID NO: 55: Amino acid sequence of heavy chain variable region of anti-RGMa neutralizing antibody 5F9
SEQ ID NO: 56: Amino acid sequence of light chain variable region of anti-RGMa neutralizing antibody 8D1
SEQ ID NO: 57: Amino acid sequence of heavy chain variable region of anti-RGMa neutralizing antibody 8D1
SEQ ID NO: 58: Amino acid sequence of light chain variable region of anti-RGMa neutralizing antibody AE12-1
SEQ ID NO: 59: Amino acid sequence of heavy chain variable region of anti-RGMa neutralizing antibody AE12-1
SEQ ID NO: 60: Amino acid sequence of light chain variable region of anti-RGMa neutralizing antibody AE12-1Y
SEQ ID NO: 61: Amino acid sequence of light chain of anti-RGMa neutralizing antibody AE12-1Y-QL (Elezanumab)
SEQ ID NO: 62: Amino acid sequence of heavy chain of anti-RGMa neutralizing antibody AE12-1Y-QL (Elezanumab)

### Industrial Applicability

RGMa inhibiting substance is expected to be useful for the prophylaxis or therapy of associated symptoms of diseases caused by a spinal cord disorder or a brain disorder, specifically spasticity and/or neurogenic bladder, and the present invention has high utility value in the pharmaceutical industry.

## Claims

1. A prophylactic or therapeutic agent for spasticity, comprising an RGMa inhibiting substance as an active ingredient.

2. The prophylactic or therapeutic agent according to claim 1, wherein the spasticity is spasticity associated with a disease caused by a spinal cord disorder or a brain disorder.

3. The prophylactic or therapeutic agent according to claim 1, wherein the spasticity is spasticity associated with spinal cord injury.

4. The prophylactic or therapeutic agent according to claim 1, wherein the spasticity is spasticity associated with HTLV-1 associated myelopathy.

5. The agent according to any one of claims 1 to 4, wherein the RGMa inhibiting substance is an anti-RGMa neutralizing antibody.

6. The agent according to claim 5, wherein the anti-RGMa neutralizing antibody is a humanized antibody.

7. The agent according to claim 5 or 6, wherein the anti-RGMa neutralizing antibody is an antibody that recognizes an amino acid sequence selected from the group consisting of SEQ ID NO: 16, SEQ ID NO: 36, SEQ ID NO: 37, SEQ ID NO: 38 and SEQ ID NO: 39.

8. The agent according to any one of claims 5 to 7, wherein the anti-RGMa neutralizing antibody is an antibody selected from the following (a) to (I):
(a) an anti-RGMa neutralizing antibody comprising a light chain variable region comprising LCDR1 comprising the amino acid sequence of SEQ ID NO: 5, LCDR2 comprising the amino acid sequence of SEQ ID NO: 6 and LCDR3 comprising the amino acid sequence of SEQ ID NO: 7, and a heavy chain variable region comprising HCDR1 comprising the amino acid sequence of SEQ ID NO: 8, HCDR2 comprising the amino acid sequence of SEQ ID NO: 9 and HCDR3 comprising the amino acid sequence of SEQ ID NO: 10;
(b) an anti-RGMa neutralizing antibody comprising a light chain variable region comprising LCDR1 comprising the amino acid sequence of SEQ ID NO: 11, LCDR2 comprising the amino acid sequence of SEQ ID NO: 12 and LCDR3 comprising the amino acid sequence of SEQ ID NO: 13, and a heavy chain variable region comprising HCDR1 comprising the amino acid sequence of SEQ ID NO: 14, HCDR2 comprising the amino acid sequence of SEQ ID NO: 15 and HCDR3 comprising the amino acid sequence of SFG;
(c) an anti-RGMa neutralizing antibody comprising a light chain variable region comprising LCDR1 comprising the amino acid sequence of SEQ ID NO: 17, LCDR2 comprising the amino acid sequence of SEQ ID NO: 18 and LCDR3 comprising the amino acid sequence of SEQ ID NO: 19, and a heavy chain variable region comprising HCDR1 comprising the amino acid sequence of SEQ ID NO: 20, HCDR2 comprising the amino acid sequence of SEQ ID NO: 21 and HCDR3 comprising the amino acid sequence of SEQ ID NO: 22;
(d) an anti-RGMa neutralizing antibody comprising a light chain variable region comprising LCDR1 comprising the amino acid sequence of SEQ ID NO: 23, LCDR2 comprising the amino acid sequence of SEQ ID NO: 24 and LCDR3 comprising the amino acid sequence of SEQ ID NO: 25, and a heavy chain variable region comprising HCDR1 comprising the amino acid sequence of SEQ ID NO: 26, HCDR2 comprising the amino acid sequence of SEQ ID NO: 27 and HCDR3 comprising the amino acid sequence of SEQ ID NO: 28;
(e) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 31, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34;
(f) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 35, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34;
(g) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 40, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34;
(h) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 41, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34;
(i) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 42, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34;
(j) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 43, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34;
(k) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 44, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34; and
(l) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 45, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34.

9. The agent according to any one of claims 5 to 8, wherein the anti-RGMa neutralizing antibody is the following (a) or (f):
(a) an anti-RGMa neutralizing antibody comprising a light chain variable region comprising LCDR1 comprising the amino acid sequence of SEQ ID NO: 5, LCDR2 comprising the amino acid sequence of SEQ ID NO: 6 and LCDR3 comprising the amino acid sequence of SEQ ID NO: 7, and a heavy chain variable region comprising HCDR1 comprising the amino acid sequence of SEQ ID NO: 8, HCDR2 comprising the amino acid sequence of SEQ ID NO: 9 and HCDR3 comprising the amino acid sequence of SEQ ID NO: 10;
(f) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 35, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34.

10. A method for prophylaxis or therapy of spasticity, comprising administering an effective amount of an RGMa inhibiting substance to a mammal in need of treatment.

11. The prophylactic or therapeutic method according to claim 10, wherein the RGMa inhibiting substance is an anti-RGMa neutralizing antibody.

12. Use of an RGMa inhibiting substance in the manufacture of a prophylactic or therapeutic agent for spasticity.

13. The use according to claim 12, wherein the RGMa inhibiting substance is an anti-RGMa neutralizing antibody.

14. A prophylactic or therapeutic agent for spasticity and neurogenic bladder, comprising an RGMa inhibiting substance as an active ingredient.

15. The prophylactic or therapeutic agent according to claim 14, wherein the spasticity and neurogenic bladder are spasticity and neurogenic bladder associated with a disease caused by a spinal cord disorder or a brain disorder.

16. The prophylactic or therapeutic agent according to claim 14, wherein the spasticity and neurogenic bladder are spasticity and neurogenic bladder associated with spinal cord injury.

17. The prophylactic or therapeutic agent according to claim 14, wherein the spasticity and neurogenic bladder are spasticity and neurogenic bladder associated with HTLV-1 associated myelopathy.

18. The agent according to any one of claims 14 to 17, wherein the RGMa inhibiting substance is an anti-RGMa neutralizing antibody.

19. The agent according to claim 18, wherein the anti-RGMa neutralizing antibody is a humanized antibody.

20. The agent according to claim 18 or 19, wherein the anti-RGMa neutralizing antibody is an antibody that recognizes an amino acid sequence selected from the group consisting of SEQ ID NO: 16, SEQ ID NO: 36, SEQ ID NO: 37, SEQ ID NO: 38 and SEQ ID NO: 39.

21. The agent according to any one of claims 18 to 20, wherein the anti-RGMa neutralizing antibody is an antibody selected from the following (a) to (I):
(a) an anti-RGMa neutralizing antibody comprising a light chain variable region comprising LCDR1 comprising the amino acid sequence of SEQ ID NO: 5, LCDR2 comprising the amino acid sequence of SEQ ID NO: 6 and LCDR3 comprising the amino acid sequence of SEQ ID NO: 7, and a heavy chain variable region comprising HCDR1 comprising the amino acid sequence of SEQ ID NO: 8, HCDR2 comprising the amino acid sequence of SEQ ID NO: 9 and HCDR3 comprising the amino acid sequence of SEQ ID NO: 10;
(b) an anti-RGMa neutralizing antibody comprising a light chain variable region comprising LCDR1 comprising the amino acid sequence of SEQ ID NO: 11, LCDR2 comprising the amino acid sequence of SEQ ID NO: 12 and LCDR3 comprising the amino acid sequence of SEQ ID NO: 13, and a heavy chain variable region comprising HCDR1 comprising the amino acid sequence of SEQ ID NO: 14, HCDR2 comprising the amino acid sequence of SEQ ID NO: 15 and HCDR3 comprising the amino acid sequence of SFG;
(c) an anti-RGMa neutralizing antibody comprising a light chain variable region comprising LCDR1 comprising the amino acid sequence of SEQ ID NO: 17, LCDR2 comprising the amino acid sequence of SEQ ID NO: 18 and LCDR3 comprising the amino acid sequence of SEQ ID NO: 19, and a heavy chain variable region comprising HCDR1 comprising the amino acid sequence of SEQ ID NO: 20, HCDR2 comprising the amino acid sequence of SEQ ID NO: 21 and HCDR3 comprising the amino acid sequence of SEQ ID NO: 22;
(d) an anti-RGMa neutralizing antibody comprising a light chain variable region comprising LCDR1 comprising the amino acid sequence of SEQ ID NO: 23, LCDR2 comprising the amino acid sequence of SEQ ID NO: 24 and LCDR3 comprising the amino acid sequence of SEQ ID NO: 25, and a heavy chain variable region comprising HCDR1 comprising the amino acid sequence of SEQ ID NO: 26, HCDR2 comprising the amino acid sequence of SEQ ID NO: 27 and HCDR3 comprising the amino acid sequence of SEQ ID NO: 28;
(e) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 31, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34;
(f) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 35, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34;
(g) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 40, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34;
(h) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 41, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34;
(i) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 42, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34;
(j) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 43, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34;
(k) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 44, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34; and
(l) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 45, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34.

22. The agent according to any one of claims 18 to 21, wherein the anti-RGMa neutralizing antibody is the following (a) or (f):
(a) an anti-RGMa neutralizing antibody comprising a light chain variable region comprising LCDR1 comprising the amino acid sequence of SEQ ID NO: 5, LCDR2 comprising the amino acid sequence of SEQ ID NO: 6 and LCDR3 comprising the amino acid sequence of SEQ ID NO: 7, and a heavy chain variable region comprising HCDR1 comprising the amino acid sequence of SEQ ID NO: 8, HCDR2 comprising the amino acid sequence of SEQ ID NO: 9 and HCDR3 comprising the amino acid sequence of SEQ ID NO: 10;
(f) an anti-RGMa neutralizing antibody comprising a light chain variable region comprising LCDR1 comprising the amino acid sequence of SEQ ID NO: 29, LCDR2 comprising the amino acid sequence of SEQ ID NO: 30 and LCDR3 comprising the amino acid sequence of SEQ ID NO: 35, and a heavy chain variable region comprising HCDR1 comprising the amino acid sequence of SEQ ID NO: 32, HCDR2 comprising the amino acid sequence of SEQ ID NO: 33 and HCDR3 comprising the amino acid sequence of SEQ ID NO: 34.

23. A method for prophylaxis or therapy of spasticity and neurogenic bladder, comprising administering an effective amount of an RGMa inhibiting substance to a mammal in need of treatment.

24. The prophylactic or therapeutic method according to claim 23, wherein the RGMa inhibiting substance is an anti-RGMa neutralizing antibody.

25. Use of an RGMa inhibiting substance in the manufacture of a prophylactic or therapeutic agent for spasticity and neurogenic bladder.

26. The use according to claim 25, wherein the RGMa inhibiting substance is an anti-RGMa neutralizing antibody.

27. A prophylactic or therapeutic agent for neurogenic bladder comprising an RGMa inhibiting substance as an active ingredient.

28. The prophylactic or therapeutic agent according to claim 27, wherein the neurogenic bladder is neurogenic bladder associated with a disease caused by a spinal cord disorder or a brain disorder.

29. The prophylactic or therapeutic agent according to claim 27, wherein the neurogenic bladder is neurogenic bladder associated with spinal cord injury.

30. The prophylactic or therapeutic agent according to claim 27, wherein the neurogenic bladder is neurogenic bladder associated with HTLV-1 associated myelopathy.

31. The agent according to any one of claims 27 to 30, wherein the RGMa inhibiting substance is an anti-RGMa neutralizing antibody.

32. The agent according to claim 31, wherein the anti-RGMa neutralizing antibody is a humanized antibody.

33. The agent according to any one of claims 31 or 32, wherein the anti-RGMa neutralizing antibody is the following (a): (a) an anti-RGMa neutralizing antibody comprising a light chain variable region comprising LCDR1 comprising the amino acid sequence of SEQ ID NO: 5, LCDR2 comprising the amino acid sequence of SEQ ID NO: 6 and LCDR3 comprising the amino acid sequence of SEQ ID NO: 7, and a heavy chain variable region comprising HCDR1 comprising the amino acid sequence of SEQ ID NO: 8, HCDR2 comprising the amino acid sequence of SEQ ID NO: 9 and HCDR3 comprising the amino acid sequence of SEQ ID NO: 10.

34. A method for prophylaxis or therapy of neurogenic bladder, comprising administering an effective amount of an RGMa inhibiting substance to a mammal in need of treatment.

35. The prophylactic or therapeutic method according to claim 34, wherein the RGMa inhibiting substance is an anti-RGMa neutralizing antibody.

36. Use of an RGMa inhibiting substance in the manufacture of a prophylactic or therapeutic agent for neurogenic bladder.

37. The use according to claim 36, wherein the RGMa inhibiting substance is an anti-RGMa neutralizing antibody.
